(19) Europäisches Patentamt
European Patent Office
Office européen des brevets

(11) **EP 3 842 037 A1**

(12) **EUROPEAN PATENT APPLICATION**

(43) Date of publication:
**30.06.2021 Bulletin 2021/26**

(21) Application number: **20211291.8**

(22) Date of filing: **13.08.2009**

(51) Int Cl.:
***A61K 9/20*** (2006.01) ***A61K 9/16*** (2006.01)
***A61P 3/00*** (2006.01) ***A61P 1/10*** (2006.01)
***A61P 3/06*** (2006.01) ***A61P 3/10*** (2006.01)
***A61K 9/28*** (2006.01) ***A61P 1/16*** (2006.01)
***A61P 11/00*** (2006.01) ***A61P 11/02*** (2006.01)
***A61K 31/47*** (2006.01) ***A61P 7/00*** (2006.01)
***A61P 7/10*** (2006.01) ***A61P 5/50*** (2006.01)
***A61P 5/14*** (2006.01) ***A61P 5/18*** (2006.01)
***A61P 37/08*** (2006.01) ***A61P 5/06*** (2006.01)
***A61P 27/02*** (2006.01) ***A61P 35/00*** (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **13.08.2008 US 88704 P**
**14.08.2008 US 88801 P**
**19.08.2008 US 90096 P**
**21.01.2009 US 146163 P**
**27.05.2009 US 181527 P**
**02.06.2009 US 183345 P**

(62) Document number(s) of the earlier application(s) in accordance with Art. 76 EPC:
**17204189.9 / 3 345 625**
**09789125.3 / 2 328 618**

(71) Applicant: **Vertex Pharmaceuticals Incorporated**
**Boston, MA 02210 (US)**

(72) Inventors:
- **ROWE, William**
  **Boston, MA 02210 (US)**
- **HURTER, Patricia**
  **Harvard, MA 01451 (US)**
- **YOUNG, Christopher**
  **Sudbury, MA 01776 (US)**
- **DINEHART, Kirk**
  **Holliston, MA 01746 (US)**
- **VERWIJS, Marinus Jacobus**
  **Sudbury, MA 01776 (US)**
- **OVERHOFF, Kirk**
  **Lynn, MA 01902 (US)**
- **GROOTENHUIS, Peter D. J.**
  **Del Mar, CA 92104 (US)**
- **BOTFIELD, Martyn**
  **Concord, MA 01742 (US)**
- **GROSSI, Alfredo**
  **Somervile, MA 02145 (US)**

(74) Representative: **Oates, Edward Christopher**
**Carpmaels & Ransford LLP**
**One Southampton Row**
**London WC1B 5HA (GB)**

Remarks:
•This application was filed on 02.12.2020 as a divisional application to the application mentioned under INID code 62.
•Claims filed after the date of filing of the application / after the date of receipt of the divisional application (Rule 68(4) EPC).

# (54) PHARMACEUTICAL COMPOSITION AND ADMINISTRATIONS THEREOF

(57) The present invention relates to pharmaceutical compositions comprising a solid dispersion of N-[2,4-Bis(1,1-dimethylethyl)-5-hydroxyphenyl]-1,4-dihydro-4-oxoquinoline-3-carboxamide, methods of manufacturing pharmaceutical compositions of the present invention, and methods of administering pharmaceutical compositions of the present invention.



Printed by Jouve, 75001 PARIS (FR)

## Description

CROSS-REFERENCE TO RELATED APPLICATIONS

This Application claims priority to USSN 61/088,704 filed on August 13, 2008, USSN 61/088,801, filed on August 14, 2008, USSN 61/090,096, filed on August 19, 2008, USSN 61/146,163, filed on January 21, 2009, USSN 61/181,527, filed on May 27, 2009, and USSN 61/183,345, filed on June 2, 2009, each of which is incorporated by reference in their entirety.

FIELD OF THE INVENTION

The present invention relates to pharmaceutical compositions comprising a solid dispersion of N-[2,4-Bis(1,1-dimethylethyl)-5-hydroxyphenyl]-1,4-dihydro-4-oxoquinoline-3-carboxamide, and methods of manufacturing and administering pharmaceutical compositions comprising N-[2,4-Bis(1,1-dimethylethyl)-5-hydroxyphenyl]-1,4-dihydro-4-oxoquinoline-3-carboxamide.

BACKGROUND

Cystic fibrosis (CF) is a recessive genetic disease that affects approximately 30,000 children and adults in the United States and approximately 30,000 children and adults in Europe. Despite progress in the treatment of CF, there is no cure.

CF is caused by mutations in the cystic fibrosis transmembrane conductance regulator (CFTR) gene that encodes an epithelial chloride ion channel responsible for aiding in the regulation of salt and water absorption and secretion in various tissues. Small molecule drugs, known as potentiators that increase the probability of CFTR channel opening represent one potential therapeutic strategy to treat CF.

Specifically, CFTR is a cAMP/ATP-mediated anion channel that is expressed in a variety of cells types, including absorptive and secretory epithelia cells, where it regulates anion flux across the membrane, as well as the activity of other ion channels and proteins. In epithelia cells, normal functioning of CFTR is critical for the maintenance of electrolyte transport throughout the body, including respiratory and digestive tissue. CFTR is composed of approximately 1480 amino acids that encode a protein made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

The gene encoding CFTR has been identified and sequenced (See Gregory, R. J. et al. (1990) Nature 347:382-386; Rich, D. P. et al. (1990) Nature 347:358-362), (Riordan, J. R. et al. (1989) Science 245:1066-1073). A defect in this gene causes mutations in CFTR resulting in cystic fibrosis ("CF"), the most common fatal genetic disease in humans. Cystic fibrosis affects approximately one in every 2,500 infants in the United States. Within the general United States population, up to 10 million people carry a single copy of the defective gene without apparent ill effects. In contrast, individuals with two copies of the CF associated gene suffer from the debilitating and fatal effects of CF, including chronic lung disease.

In patients with CF, mutations in CFTR endogenously expressed in respiratory epithelia leads to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, results in death. In addition, the majority of males with cystic fibrosis are infertile and fertility is decreased among females with cystic fibrosis. In contrast to the severe effects of two copies of the CF associated gene, individuals with a single copy of the CF associated gene exhibit increased resistance to cholera and to dehydration resulting from diarrhea - perhaps explaining the relatively high frequency of the CF gene within the population.

Sequence analysis of the CFTR gene of CF chromosomes has revealed a variety of disease causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, > 1000 disease causing mutations in the CF gene have been identified (http://www.genet.sickkids.on.ca/cftr/). The most prevalent mutation is a deletion of phenylalanine at position 508 of the CFTR amino acid sequence, and is commonly referred to as ΔF508-CFTR. This mutation occurs in approximately 70% of the cases of cystic fibrosis and is associated with a severe disease.

The deletion of residue 508 in ΔF508-CFTR prevents the nascent protein from folding correctly. This results in the inability of the mutant protein to exit the ER, and traffic to the plasma membrane. As a result, the number of channels present in the membrane is far less than observed in cells expressing wild-type CFTR. In addition to impaired trafficking, the mutation results in defective channel gating. Together, the reduced number of channels in the membrane and the

defective gating lead to reduced anion transport across epithelia leading to defective ion and fluid transport. (Quinton, P. M. (1990), FASEB J. 4: 2709-2727). Studies have shown, however, that the reduced numbers of ΔF508-CFTR in the membrane are functional, albeit less than wild-type CFTR. (Dalemans et al. (1991), Nature Lond. 354: 526-528; Denning et al., supra; Pasyk and Foskett (1995), J. Cell. Biochem. 270: 12347-50). In addition to ΔF508-CFTR, other disease causing mutations in CFTR that result in defective trafficking, synthesis, and/or channel gating could be up- or down-regulated to alter anion secretion and modify disease progression and/or severity.

Although CFTR transports a variety of molecules in addition to anions, it is clear that this role (the transport of anions) represents one element in an important mechanism of transporting ions and water across the epithelium. The other elements include the epithelial Na+ channel, ENaC, Na+/2Cl-/K+ co-transporter, Na+-K+-ATPase pump and the basolateral membrane K+ channels, that are responsible for the uptake of chloride into the cell.

These elements work together to achieve directional transport across the epithelium via their selective expression and localization within the cell. Chloride absorption takes place by the coordinated activity of ENaC and CFTR present on the apical membrane and the Na+-K+-ATPase pump and Cl ion channels expressed on the basolateral surface of the cell. Secondary active transport of chloride from the luminal side leads to the accumulation of intracellular chloride, which can then passively leave the cell via Cl- channels, resulting in a vectorial transport. Arrangement of Na+/2Cl-/K+ co-transporter, Na+-K+-ATPase pump and the basolateral membrane K+ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride.

As discussed above, it is believed that the deletion of residue 508 in ΔF508-CFTR prevents the nascent protein from folding correctly, resulting in the inability of this mutant protein to exit the ER, and traffic to the plasma membrane. As a result, insufficient amounts of the mature protein are present at the plasma membrane and chloride transport within epithelial tissues is significantly reduced. In fact, this cellular phenomenon of defective ER processing of ABC transporters by the ER machinery has been shown to be the underlying basis not only for CF disease, but for a wide range of other isolated and inherited diseases.

N-[2,4-bis(1,1-dimethylethyl)-5-hydroxyphenyl]-1,4-dihydro-4-oxoquinoline-3-carboxamide is a potent and selective CFTR potentiator of wild-type and mutant (including e.g., ΔF508, R117H, and G551D) forms of human CFTR. N-[2,4-bis(1,1-dimethylethyl)-5-hydroxyphenyl]-1,4-dihydro-4-oxoquinoline-3-carboxamide is useful for treatment of adult patients with cystic fibrosis and at least one G551D-CFTR allele.

Accordingly, there is a need for stable bioavailable pharmaceutical compositions of N-[2,4-bis(1,1-dimethylethyl)-5-hydroxyphenyl]-1,4-dihydro-4-oxoquinoline-3-carboxamide useful for treating patients suffering from CF and methods of administering the same.

## SUMMARY OF THE INVENTION

In general, the invention relates to pharmaceutical compositions comprising a solid dispersion of N-[2,4-bis(1,1-dimethylethyl)-5-hydroxyphenyl]-1,4-dihydro-4-oxoquinoline-3-carboxamide ("Compound 1"). The pharmaceutical compositions may also include one or more of the following excipients: a filler, a disintegrant, a glidant, a lubricant, a binder, and a surfactant.

In one embodiment, the present invention provides a pharmaceutical composition comprising a solid dispersion of amorphous Compound 1, wherein the solid dispersion comprises about 25 mg of amorphous Compound 1. In certain embodiments, the solid dispersion comprises about 25 mg of substantially amorphous Compound 1.

In one embodiment, the present invention provides a pharmaceutical composition comprising a solid dispersion of amorphous Compound 1, wherein the solid dispersion comprises about 50 mg of amorphous Compound 1. In certain embodiments, the solid dispersion comprises about 50 mg of substantially amorphous Compound 1.

In one embodiment, the present invention provides a pharmaceutical composition comprising a solid dispersion of amorphous Compound 1, wherein the solid dispersion comprises about 75 mg of amorphous Compound 1. In certain embodiments, the solid dispersion comprises about 75 mg of substantially amorphous Compound 1.

In one embodiment, the present invention provides a pharmaceutical composition comprising a solid dispersion of amorphous Compound 1, wherein the solid dispersion comprises about 100 mg of amorphous Compound 1. In certain embodiments, the solid dispersion comprises about 100 mg of substantially amorphous Compound 1.

In one embodiment, the present invention provides a pharmaceutical composition comprising a solid dispersion of amorphous Compound 1, wherein the solid dispersion comprises about 150 mg of amorphous Compound 1. In certain embodiments, the solid dispersion comprises about 150 mg of substantially amorphous Compound 1.

In one embodiment, the present invention provides a pharmaceutical composition comprising a solid dispersion of amorphous Compound 1, wherein the solid dispersion comprises about 250 mg of amorphous Compound 1. In certain embodiments, the solid dispersion comprises about 250 mg of substantially amorphous Compound 1.

In one aspect, the solid form of Compound 1 in the pharmaceutical composition is a solid dispersion comprising

substantially amorphous or amorphous Compound 1 and a polymer, such as hydroxypropylmethylcellulose (HPMC), hydroxypropylmethylcellulose acetate succinate (HPMCAS), vinylpyrrolidone/vinyl acetate copolymer (PVP/VA), polyvinylpyrrolidone (PVP), methacrylic acid/methacrylate copolymers, hydroxypropyl cellulose (HPC), or any combination thereof. Embodiments of this aspect include one or more of the following: The solid dispersion is a powder having mean particle diameter of greater than about 5 $\mu$m or the solid dispersion has a bulk density of about 0.10 g/cc or greater.

In some instances, the solid dispersion has a concentration of at least 20 wt% of Compound 1, by weight of the solid dispersion. In other instances, the solid dispersion comprises 80 wt% or less of HPMCAS or PVP/VA. Some solid dispersions comprise from about 40 wt% to about 60 wt% of substantially amorphous or amorphous Compound 1 by weight of the solid dispersion and from about 60 wt% to about 40 wt% of polymer by weight of the solid dispersion. Other solid dispersions comprise from about 65 wt% to about 95 wt% of substantially amorphous or amorphous Compound 1 by weight of the solid dispersion and from about 45 wt% to about 5 wt% of polymer by weight of the solid dispersion.

Solid dispersions can also optionally comprise additives such as a surfactant (e.g., sodium lauryl sulfate (SLS)), which can be present in a concentration of less than 10 wt% of surfactant by weight of solid dispersion.

Still other solid dispersions comprise from about 45 wt% to about 85 wt% of substantially amorphous or amorphous Compound 1, from about 0.45 wt% to about 0.55 wt% of SLS, and from about 14.45 wt% to about 55.55 wt% of HPMCAS or PVP/VA by weight of the solid dispersion.

In still further embodiments, the pharmaceutical compositions also comprise a filler (e.g., lactose, sorbitol, celluloses, calcium phosphates, starches, sugars (e.g., mannitol, sucrose, or the like) or any combination thereof) in concentrations of at least about 10 wt% by weight of the composition; a disintegrant (e.g., sodium croscarmellose, sodium starch glycolate, or a combination thereof) in concentrations of about 10 wt% or less by weight of the composition; a surfactant (e.g., sodium lauryl sulfate, sodium stearyl fumarate (SSF), polyoxyethylene 20 sorbitan mono-oleate, or any combination thereof) in concentrations of about 10 wt% or less by weight of the composition; a binder (e.g., microcrystalline cellulose, dibasic calcium phosphate, sucrose, corn (maize) starch, modified cellulose (e.g., hydroxymethyl cellulose), or any combination thereof) in concentrations of at least about 1 wt% by weight of the composition; a glidant (e.g., colloidal silicon dioxide, talc, or a combination thereof) in concentrations of about 2 wt% or less by weight of the composition; and a lubricant (e.g., magnesium stearate, stearic acid, hydrogenated oil, sodium stearyl fumarate, or any combination thereof) in concentrations of about 2 wt% or less by weight of the composition.

Such pharmaceutical compositions can optionally comprise one or more colorants, fragrances, and/or flavors to enhance its visual appeal, taste, and scent.

Another aspect of the present invention provides a pharmaceutical composition consisting of a tablet that comprises a solid dispersion, a filler, a disintegrant, a surfactant, a binder, a glidant, and a lubricant, wherein the tablet has a dissolution of at least about 50% in about 30 minutes, and the solid dispersion comprises substantially amorphous Compound 1. As noted below, dissolution is measured with a standard USP Type II apparatus that employs a dissolution media of 0.6% sodium lauryl sulfate dissolved in 900 mL of DI water (or a volume of media having the same ratio of SLS to DI water) at a temperature of about 37 °C. A single experimental tablet is tested in each test vessel of the apparatus. Dissolution can also be measured with a standard USP Type II apparatus that employs a dissolution media of 0.7% sodium lauryl sulfate dissolved in 900 mL of 50 mM sodium phosphate buffer (pH 6.8) at a temperature of about 37 °C. Dissolution can also be measured with a standard USP Type II apparatus that employs a dissolution media of 0.5% sodium lauryl sulfate dissolved in 900 mL of 50 mM sodium phosphate buffer (pH 6.8) at a temperature of about 37 °C. A single experimental tablet is tested in each test vessel of the apparatus.

Another aspect of the present invention provides a pharmaceutical composition consisting of a tablet that comprises a solid dispersion comprising amorphous or substantially amorphous Compound 1 and HPMCAS or PVP/VA; and, a filler, a disintegrant, a surfactant, a binder, a glidant, and a lubricant, wherein the tablet has a hardness of at least about 5 Kp.

In yet another aspect, the tablets described herein are coated.

In another aspect, the coated tablets described herein are colored.

In still another aspect, the colored, coated tablets include text or images. For instance, the text or images can be printed on the colored, coated tablet. In still other aspects, the colored, coated tablets include about 3 wt% of a film coating comprising a blue colorant, such as OPADRY® II. In some embodiments, the colored tablets can be labeled with a logo and text indicating the strength of the active ingredient in the tablet using a black ink, such as Opacode® WB or Opacode® S-1-17823. In still further embodiments, the colored, coated tablets are coated with a colorant, waxed, and then labeled with a logo, other image, and/or text using a suitable ink. In some embodiments, the tablets are coated with about 3 wt% of colorant, and waxed with Carnauba wax powder weighed out in the amount of about 0.01% w/w of the starting tablet core weight. The waxed tablets can be labeled with a logo and text indicating the strength of the active ingredient in the tablet using a suitable ink.

Another aspect of the present invention provides a method of producing a pharmaceutical composition comprising the steps of providing an admixture of a solid dispersion of amorphous Compound 1, a binder, a glidant, a surfactant, a lubricant, a disintegrant, and a filler, and compressing the admixture into a tablet having a dissolution of at

least about 50% in about 30 minutes. In one example, the admixture is compressed to a hardness of at least about 5 Kp.

Another aspect of the present invention provides a method of producing a pharmaceutical composition comprising the steps of providing an admixture of a solid dispersion of amorphous Compound 1, a binder, a glidant, a surfactant, a lubricant, a disintegrant, and a filler, and compressing the admixture into a tablet having a dissolution of at least about 70% in about 30 minutes.

Another aspect of the present invention provides a method of administering a pharmaceutical composition by orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion comprises at least about 25 mg of substantially amorphous or amorphous Compound 1. In some embodiments, the tablet is orally administered to the patient once per day. Other tablets useful in this method comprise a solid dispersion containing at least about 50 mg of substantially amorphous or amorphous Compound 1. Some tablets useful in this method comprise a solid dispersion containing at least about 75 mg of substantially amorphous or amorphous Compound 1. Other tablets useful in this method comprise a solid dispersion containing at least about 100 mg of substantially amorphous or amorphous Compound 1. Yet other tablets useful in this method comprise a solid dispersion containing at least about 150 mg of substantially amorphous or amorphous Compound 1. In another method, the administration comprises orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion contains at least about 250 mg of substantially amorphous or amorphous Compound 1. In some embodiments, the present invention provides for a method of orally administering the pharmaceutical compositions described herein at least once a day. In other embodiments, the present invention provides for a method of orally administering the pharmaceutical composition described herein once a day. In some embodiments, the present invention provides for a method of orally administering the pharmaceutical composition described herein twice a day.

In one aspect, the invention also provides a method of treating or lessening the severity of a disease in a patient comprising administering to said patient one of the compositions as defined herein, and said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, Sjogren's disease, Osteoporosis, Osteopenia, Gorham's Syndrome, chloride channelopathies such as myotonia congenita (Thomson and Becker forms), Bartter's syndrome type III, Dent's disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

## BRIEF DESCRIPTION OF THE FIGURES

Figure 1 presents a graphical illustration of the dissolution profiles of exemplary tablets according to the present invention.

This figure is presented by way of example and is not intended to be limiting.

## DETAILED DESCRIPTION

The present invention provides a pharmaceutical composition comprising a solid dispersion of N-[2,4-bis(1,1-dimethylethyl)-5-hydroxyphenyl]-1,4-dihydro-4-oxoquinoline-3-carboxamide, a method of manufacturing a pharmaceutical composition comprising N-[2,4-bis(1,1-dimethylethyl)-5-hydroxyphenyl]-1,4-dihydro-4-oxoquinoline-3-carboxamide, and a method of administering a pharmaceutical composition comprising a solid form of N-[2,4-bis(1,1-dimethylethyl)-5-hydroxyphenyl]-1,4-dihydro-4-oxoquinoline-3 -carboxamide.

I. DEFINITIONS

As used herein, the term "active pharmaceutical ingredient" or "API" refers to a biologically active compound. Exemplary APIs include a CF potentiator (e.g., N-[2,4-bis(1,1-dimethylethyl)-5-hydroxyphenyl]-1,4-dihydro-4-oxoquinoline-3-carboxamide).

As used herein, the term "Compound 1" is used interchangeably with "N-[2,4-bis(1,1-dimethylethyl)-5-hydroxyphenyl]-1,4-dihydro-4-oxoquinoline-3-carboxamide", which has the following structure:

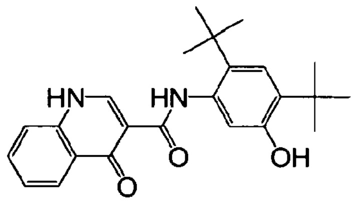

"Compound 1" also means tautomeric forms such as:

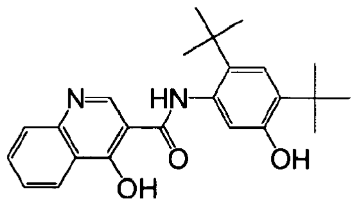

As used herein, the term "amorphous" refers to a solid material having no long range order in the position of its molecules. Amorphous solids are generally supercooled liquids in which the molecules are arranged in a random manner so that there is no well-defined arrangement, e.g., molecular packing, and no long range order. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. See, US 2004/0006237 for a comparison of XRPDs of an amorphous material and crystalline material.

As used herein, the term "substantially amorphous" refers to a solid material having little or no long range order in the position of its molecules. For example, substantially amorphous materials have less than about 15% crystallinity (e.g., less than about 10% crystallinity or less than about 5% crystallinity). It is also noted that the term 'substantially amorphous' includes the descriptor, 'amorphous', which refers to materials having no (0%) crystallinity.

As used herein, the term "dispersion" refers to a disperse system in which one substance, the dispersed phase, is distributed, in discrete units, throughout a second substance (the continuous phase or vehicle). The size of the dispersed phase can vary considerably (e.g. single molecules, colloidal particles of nanometer dimension, to multiple microns in size). In general, the dispersed phases can be solids, liquids, or gases. In the case of a solid dispersion, the dispersed and continuous phases are both solids. In pharmaceutical applications, a solid dispersion can include: an amorphous drug in an amorphous polymer; an amorphous drug in crystalline polymer; a crystalline drug in an amorphous polymer; or a crystalline drug in crystalline polymer. In this invention, a solid dispersion can include an amorphous drug in an amorphous polymer or an amorphous drug in crystalline polymer. In some embodiments, a solid dispersion includes the polymer constituting the dispersed phase, and the drug constitutes the continuous phase. Or, a solid dispersion includes the drug constituting the dispersed phase, and the polymer constitutes the continuous phase.

As used herein, the term "solid dispersion" generally refers to a solid dispersion of two or more components, usually one or more drugs (e.g., one drug (e.g., Compound 1)) and polymer, but possibly containing other components such as surfactants or other pharmaceutical excipients, where the drug(s) (e.g., Compound 1) is substantially amorphous (e.g., having about 15% or less (e.g., about 10% or less, or about 5% or less)) of crystalline drug (e.g., N-[2,4-bis(1,1-dimethylethyl)-5-hydroxyphenyl]-1,4-dihydro-4-oxoquinoline-3-carboxamide) or amorphous (i.e., having no crystalline drug), and the physical stability and/or dissolution and/or solubility of the substantially amorphous or amorphous drug is enhanced by the other components. Solid dispersions typically include a compound dispersed in an appropriate carrier medium, such as a solid state carrier. For example, a carrier comprises a polymer (e.g., a water-soluble polymer or a partially water-soluble polymer) and can include optional excipients such as functional excipients (e.g., one or more surfactants) or nonfunctional excipients (e.g., one or more fillers). Another exemplary solid dispersion is a co-precipitate

or a co-melt of N-[2,4-bis(1,1-dimethylethyl)-5-hydroxyphenyl]-1,4-dihydro-4-oxoquinoline-3-carboxamide with at least one polymer.

A "Co-precipitate" is a product after dissolving a drug and a polymer in a solvent or solvent mixture followed by the removal of the solvent or solvent mixture. Sometimes the polymer can be suspended in the solvent or solvent mixture. The solvent or solvent mixture includes organic solvents and supercritical fluids. A "co-melt" is a product after heating a drug and a polymer to melt, optionally in the presence of a solvent or solvent mixture, followed by mixing, removal of at least a portion of the solvent if applicable, and cooling to room temperature at a selected rate.

As used herein, "crystallinity" refers to the degree of structural order in a solid. For example, Compound 1, which is substantially amorphous, has less than about 15% crystallinity, or its solid state structure is less than about 15% crystalline. In another example, Compound 1, which is amorphous, has zero (0%) crystallinity.

As used herein, a "CF potentiator" refers to a compound that exhibits biological activity characterized by increasing gating functionality of the mutant CFTR protein present in the cell surface to approximately wild type levels.

As used herein, an "excipient" is an inactive ingredient in a pharmaceutical composition. Examples of excipients include fillers or diluents, surfactants, binders, glidants, lubricants, disintegrants, and the like.

As used herein, a "disintegrant" is an excipient that hydrates a pharmaceutical composition and aids in tablet dispersion. Examples of disintegrants include sodium croscarmellose and/or sodium starch glycolate.

As used herein, a "diluent" or "filler" is an excipient that adds bulkiness to a pharmaceutical composition. Examples of fillers include lactose, sorbitol, celluloses, calcium phosphates, starches, sugars (e.g., mannitol, sucrose, or the like) or any combination thereof.

As used herein, a "surfactant" is an excipient that imparts pharmaceutical compositions with enhanced solubility and/or wetability. Examples of surfactants include sodium lauryl sulfate (SLS), sodium stearyl fumarate (SSF), polyoxyethylene 20 sorbitan mono-oleate (e.g., Tween™), or any combination thereof.

As used herein, a "binder" is an excipient that imparts a pharmaceutical composition with enhanced cohesion or tensile strength (e.g., hardness). Examples of binders include dibasic calcium phosphate, sucrose, corn (maize) starch, microcrystalline cellulose, and modified cellulose (e.g., hydroxymethyl cellulose).

As used herein, a "glidant" is an excipient that imparts a pharmaceutical compositions with enhanced flow properties. Examples of glidants include colloidal silica and/or talc.

As used herein, a "colorant" is an excipient that imparts a pharmaceutical composition with a desired color. Examples of colorants include commercially available pigments such as FD&C Blue # 1 Aluminum Lake, FD&C Blue #2, other FD&C Blue colors, titanium dioxide, iron oxide, and/or combinations thereof.

As used herein, a "lubricant" is an excipient that is added to pharmaceutical compositions that are pressed into tablets. The lubricant aids in compaction of granules into tablets and ejection of a tablet of a pharmaceutical composition from a die press. Examples of lubricants include magnesium stearate, stearic acid (stearin), hydrogenated oil, sodium stearyl fumarate, or any combination thereof.

As used herein, "friability" refers to the property of a tablet to remain intact and withhold its form despite an external force of pressure. Friability can be quantified using the mathematical expression presented in equation 1:

$$\%\, friabiliy = 100 \times \frac{(W_0 - W_f)}{W_0} \qquad (1)$$

wherein $W_0$ is the original weight of the tablet and $W_f$ is the final weight of the tablet after it is put through the friabilator.

Friability is measured using a standard USP testing apparatus that tumbles experimental tablets for 100 revolutions. Some tablets of the present invention have a friability of less than about 1% (e.g., less than about 0.75%, less than about 0.50%, or less than about 0.30%)

As used herein, "mean particle diameter" is the average particle diameter as measured using techniques such as laser light scattering, image analysis, or sieve analysis.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed.

**II.** PHARMACEUTICAL COMPOSITION

In one aspect, the present invention provides a pharmaceutical composition comprising a CF potentiator API (e.g., a solid dispersion of Compound 1).

In one embodiment, the present invention provides a pharmaceutical composition comprising a solid dispersion of substantially amorphous Compound 1, wherein the solid dispersion comprises about 25 mg of substantially amorphous

Compound 1.

In one embodiment, the present invention provides a pharmaceutical composition comprising a solid dispersion of substantially amorphous Compound 1, wherein the solid dispersion comprises about 50 mg of substantially amorphous Compound 1.

In one embodiment, the present invention provides a pharmaceutical composition comprising a solid dispersion of substantially amorphous Compound 1, wherein the solid dispersion comprises about 75 mg of substantially amorphous Compound 1.

In one embodiment, the present invention provides a pharmaceutical composition comprising a solid dispersion of substantially amorphous Compound 1, wherein the solid dispersion comprises about 100 mg of substantially amorphous Compound 1.

In one embodiment, the present invention provides a pharmaceutical composition comprising a solid dispersion of substantially amorphous Compound 1, wherein the solid dispersion comprises about 150 mg of substantially amorphous Compound 1.

In one embodiment, the present invention provides a pharmaceutical composition comprising a solid dispersion of substantially amorphous Compound 1, wherein the solid dispersion comprises about 250 mg of substantially amorphous Compound 1.

In one embodiment, the present invention provides a pharmaceutical composition comprising a solid dispersion of amorphous Compound 1, wherein the solid dispersion comprises about 25 mg of amorphous Compound 1.

In one embodiment, the present invention provides a pharmaceutical composition comprising a solid dispersion of amorphous Compound 1, wherein the solid dispersion comprises about 50 mg of amorphous Compound 1.

In one embodiment, the present invention provides a pharmaceutical composition comprising a solid dispersion of amorphous Compound 1, wherein the solid dispersion comprises about 75 mg of amorphous Compound 1.

In one embodiment, the present invention provides a pharmaceutical composition comprising a solid dispersion of amorphous Compound 1, wherein the solid dispersion comprises about 100 mg of amorphous Compound 1.

In one embodiment, the present invention provides a pharmaceutical composition comprising a solid dispersion of amorphous Compound 1, wherein the solid dispersion comprises about 150 mg of amorphous Compound 1.

In one embodiment, the present invention provides a pharmaceutical composition comprising a solid dispersion of amorphous Compound 1, wherein the solid dispersion comprises about 250 mg of amorphous Compound 1.

Another aspect of the present invention provides a pharmaceutical composition comprising a solid dispersion of Compound 1 in which the solid dispersion comprises a polymer.

In one embodiment, the present invention provides a pharmaceutical composition comprising a solid dispersion of substantially amorphous Compound 1 and HPMCAS, wherein the solid dispersion comprises about 25 mg of substantially amorphous Compound 1.

In one embodiment, the present invention provides a pharmaceutical composition comprising a solid dispersion of substantially amorphous Compound 1 and HPMCAS, wherein the solid dispersion comprises about 50 mg of substantially amorphous Compound 1.

In one embodiment, the present invention provides a pharmaceutical composition comprising a solid dispersion of substantially amorphous Compound 1 and HPMCAS, wherein the solid dispersion comprises about 75 mg of substantially amorphous Compound 1.

In one embodiment, the present invention provides a pharmaceutical composition comprising a solid dispersion of substantially amorphous Compound 1 and HPMCAS, wherein the solid dispersion comprises about 100 mg of substantially amorphous Compound 1.

In one embodiment, the present invention provides a pharmaceutical composition comprising a solid dispersion of substantially amorphous Compound 1 and HPMCAS, wherein the solid dispersion comprises about 150 mg of substantially amorphous Compound 1.

In one embodiment, the present invention provides a pharmaceutical composition comprising a solid dispersion of substantially amorphous Compound 1 and HPMCAS, wherein the solid dispersion comprises about 250 mg of substantially amorphous Compound 1.

In one embodiment, the present invention provides a pharmaceutical composition comprising a solid dispersion of amorphous Compound 1 and HPMCAS, wherein the solid dispersion comprises about 25 mg of amorphous Compound 1.

In one embodiment, the present invention provides a pharmaceutical composition comprising a solid dispersion of amorphous Compound 1 and HPMCAS, wherein the solid dispersion comprises about 50 mg of amorphous Compound 1.

In one embodiment, the present invention provides a pharmaceutical composition comprising a solid dispersion of amorphous Compound 1 and HPMCAS, wherein the solid dispersion comprises about 75 mg of amorphous Compound 1.

In one embodiment, the present invention provides a pharmaceutical composition comprising a solid dispersion

of amorphous Compound 1 and HPMCAS, wherein the solid dispersion comprises about 100 mg of amorphous Compound 1.

In one embodiment, the present invention provides a pharmaceutical composition comprising a solid dispersion of amorphous Compound 1 and HPMCAS, wherein the solid dispersion comprises about 150 mg of amorphous Compound 1.

In one embodiment, the present invention provides a pharmaceutical composition comprising a solid dispersion of amorphous Compound 1 and HPMCAS, wherein the solid dispersion comprises about 250 mg of amorphous Compound 1.

In one embodiment, the present invention provides a pharmaceutical composition comprising a solid dispersion of substantially amorphous Compound 1 and PVP/VA, wherein the solid dispersion comprises about 25 mg of substantially amorphous Compound 1.

In one embodiment, the present invention provides a pharmaceutical composition comprising a solid dispersion of substantially amorphous Compound 1 and PVP/VA, wherein the solid dispersion comprises about 50 mg of substantially amorphous Compound 1.

In one embodiment, the present invention provides a pharmaceutical composition comprising a solid dispersion of substantially amorphous Compound 1 and PVP/VA, wherein the solid dispersion comprises about 75 mg of substantially amorphous Compound 1.

In one embodiment, the present invention provides a pharmaceutical composition comprising a solid dispersion of substantially amorphous Compound 1 and PVP/VA, wherein the solid dispersion comprises about 100 mg of substantially amorphous Compound 1.

In one embodiment, the present invention provides a pharmaceutical composition comprising a solid dispersion of substantially amorphous Compound 1 and PVP/VA, wherein the solid dispersion comprises about 150 mg of substantially amorphous Compound 1.

In one embodiment, the present invention provides a pharmaceutical composition comprising a solid dispersion of substantially amorphous Compound 1 and PVP/VA, wherein the solid dispersion comprises about 250 mg of substantially amorphous Compound 1.

In one embodiment, the present invention provides a pharmaceutical composition comprising a solid dispersion of amorphous Compound 1 and PVP/VA, wherein the solid dispersion comprises about 25 mg of amorphous Compound 1.

In one embodiment, the present invention provides a pharmaceutical composition comprising a solid dispersion of amorphous Compound 1 and PVP/VA, wherein the solid dispersion comprises about 50 mg of amorphous Compound 1.

In one embodiment, the present invention provides a pharmaceutical composition comprising a solid dispersion of amorphous Compound 1 and PVP/VA, wherein the solid dispersion comprises about 75 mg of amorphous Compound 1.

In one embodiment, the present invention provides a pharmaceutical composition comprising a solid dispersion of amorphous Compound 1 and PVP/VA, wherein the solid dispersion comprises about 100 mg of amorphous Compound 1.

In one embodiment, the present invention provides a pharmaceutical composition comprising a solid dispersion of amorphous Compound 1 and PVP/VA, wherein the solid dispersion comprises about 150 mg of amorphous Compound 1.

In one embodiment, the present invention provides a pharmaceutical composition comprising a solid dispersion of amorphous Compound 1 and PVP/VA, wherein the solid dispersion comprises about 250 mg of amorphous Compound 1.

One aspect of the present invention provides a pharmaceutical composition comprising a CF potentiator API (e.g., a solid dispersion of Compound 1) and other excipients (e.g., a filler, a disintegrant, a surfactant, a binder, a glidant, a colorant, a lubricant, or any combination thereof).

In one embodiment, the present invention provides a pharmaceutical composition comprising:

a. a solid dispersion of substantially amorphous Compound 1 and a polymer;
b. a filler;
c. a disintegrant;
d. a surfactant;
e. a binder;
f. a glidant; and
g. a lubricant,

wherein the solid dispersion comprises about 25 mg of substantially amorphous Compound 1.

In one embodiment, the present invention provides a pharmaceutical composition comprising:

a. a solid dispersion of substantially amorphous Compound 1 and a polymer;

b. a filler;
c. a disintegrant;
d. a surfactant;
e. a binder;
f. a glidant; and
g. a lubricant,

wherein the solid dispersion comprises about 50 mg of substantially amorphous Compound 1.

In another embodiment, the present invention provides a pharmaceutical composition comprising:

a. a solid dispersion of substantially amorphous Compound 1 and a polymer;
b. a filler;
c. a disintegrant;
d. a surfactant;
e. a binder;
f. a glidant; and
g. a lubricant,

wherein the solid dispersion comprises about 75 mg of substantially amorphous Compound 1.

In one embodiment, the present invention provides a pharmaceutical composition comprising:

a. a solid dispersion of substantially amorphous Compound 1 and a polymer;
b. a filler;
c. a disintegrant;
d. a surfactant;
e. a binder;
f. a glidant; and
g. a lubricant,

wherein the solid dispersion comprises about 100 mg of substantially amorphous Compound 1.

In one embodiment, the present invention provides a pharmaceutical composition comprising:

a. a solid dispersion of substantially amorphous Compound 1 and a polymer;
b. a filler;
c. a disintegrant;
d. a surfactant;
e. a binder;
f. a glidant; and
g. a lubricant,

wherein the solid dispersion comprises about 150 mg of substantially amorphous Compound 1.

In another embodiment, the present invention provides a pharmaceutical composition comprising:

a. a solid dispersion of substantially amorphous Compound 1 and a polymer;
b. a filler;
c. a disintegrant;
d. a surfactant;
e. a binder;
f. a glidant; and
g. a lubricant,

wherein the solid dispersion comprises about 250 mg of substantially amorphous Compound 1.

In one embodiment, the present invention provides a pharmaceutical composition comprising:

a. a solid dispersion of amorphous Compound 1 and a polymer;
b. a filler;
c. a disintegrant;
d. a surfactant;

e. a binder;
f. a glidant; and
g. a lubricant,

wherein the solid dispersion comprises about 25 mg of amorphous Compound 1.

In one embodiment, the present invention provides a pharmaceutical composition comprising:

a. a solid dispersion of amorphous Compound 1 and a polymer;
b. a filler;
c. a disintegrant;
d. a surfactant;
e. a binder;
f. a glidant; and
g. a lubricant,

wherein the solid dispersion comprises about 50 mg of amorphous Compound 1.

In another embodiment, the present invention provides a pharmaceutical composition comprising:

a. a solid dispersion of amorphous Compound 1 and a polymer;
b. a filler;
c. a disintegrant;
d. a surfactant;
e. a binder;
f. a glidant; and
g. a lubricant,

wherein the solid dispersion comprises about 75 mg of amorphous Compound 1.

In one embodiment, the present invention provides a pharmaceutical composition comprising:

a. a solid dispersion of amorphous Compound 1 and a polymer;
b. a filler;
c. a disintegrant;
d. a surfactant;
e. a binder;
f. a glidant; and
g. a lubricant,

wherein the solid dispersion comprises about 100 mg of amorphous Compound 1.

In one embodiment, the present invention provides a pharmaceutical composition comprising:

a. a solid dispersion of amorphous Compound 1 and a polymer;
b. a filler;
c. a disintegrant;
d. a surfactant;
e. a binder;
f. a glidant; and
g. a lubricant,

wherein the solid dispersion comprises about 150 mg of amorphous Compound 1.

In another embodiment, the present invention provides a pharmaceutical composition comprising:

a. a solid dispersion of amorphous Compound 1 and a polymer;
b. a filler;
c. a disintegrant;
d. a surfactant;
e. a binder;
f. a glidant; and
g. a lubricant,

wherein the solid dispersion comprises about 250 mg of amorphous Compound 1.

In one embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 25 mg of substantially amorphous Compound 1.

In one embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 50 mg of substantially amorphous Compound 1.

In another embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 75 mg of substantially amorphous Compound 1.

In one embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 100 mg of substantially amorphous Compound 1.

In one embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 150 mg of substantially amorphous Compound 1.

In another embodiment, the present invention provides a pharmaceutical composition comprising:

a. a solid dispersion of substantially amorphous Compound 1 and PVP/VA;
b. a filler;
c. a disintegrant;
d. a surfactant;
e. a binder;
f. a glidant; and
g. a lubricant,

wherein the solid dispersion comprises about 250 mg of substantially amorphous Compound 1.

In one embodiment, the present invention provides a pharmaceutical composition comprising:

a. a solid dispersion of amorphous Compound 1 and PVP/VA;
b. a filler;
c. a disintegrant;
d. a surfactant;
e. a binder;
f. a glidant; and
g. a lubricant,

wherein the solid dispersion comprises about 25 mg of amorphous Compound 1.

In one embodiment, the present invention provides a pharmaceutical composition comprising:

a. a solid dispersion of amorphous Compound 1 and PVP/VA;
b. a filler;
c. a disintegrant;
d. a surfactant;
e. a binder;
f. a glidant; and
g. a lubricant,

wherein the solid dispersion comprises about 50 mg of amorphous Compound 1.

In another embodiment, the present invention provides a pharmaceutical composition comprising:

a. a solid dispersion of amorphous Compound 1 and PVP/VA;
b. a filler;
c. a disintegrant;
d. a surfactant;
e. a binder;
f. a glidant; and
g. a lubricant,

wherein the solid dispersion comprises about 75 mg of amorphous Compound 1.

In one embodiment, the present invention provides a pharmaceutical composition comprising:

a. a solid dispersion of amorphous Compound 1 and PVP/VA;
b. a filler;
c. a disintegrant;
d. a surfactant;
e. a binder;
f. a glidant; and
g. a lubricant,

wherein the solid dispersion comprises about 100 mg of amorphous Compound 1.

In one embodiment, the present invention provides a pharmaceutical composition comprising:

a. a solid dispersion of amorphous Compound 1 and PVP/VA;
b. a filler;
c. a disintegrant;

d. a surfactant;
e. a binder;
f. a glidant; and
g. a lubricant,

wherein the solid dispersion comprises about 150 mg of amorphous Compound 1.

In another embodiment, the present invention provides a pharmaceutical composition comprising:

a. a solid dispersion of amorphous Compound 1 and PVP/VA;
b. a filler;
c. a disintegrant;
d. a surfactant;
e. a binder;
f. a glidant; and
g. a lubricant,

wherein the solid dispersion comprises about 250 mg of amorphous Compound 1.

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 25 mg of substantially amorphous Compound 1.

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 50 mg of substantially amorphous Compound 1.

In another embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 75 mg of substantially amorphous Compound 1.

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and

g. a lubricant,

wherein the solid dispersion comprises about 100 mg of substantially amorphous Compound 1.

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 150 mg of substantially amorphous Compound 1.

In another embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 250 mg of substantially amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 25 mg of amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 50 mg of amorphous Compound 1.

In another embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 75 mg of amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 100 mg of amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 150 mg of amorphous Compound 1.

In another embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 250 mg of amorphous Compound 1.

In one embodiment, the pharmaceutical composition comprises a solid dispersion, a filler, a disintegrant, a surfactant, a binder, a glidant, and a lubricant, wherein the solid dispersion comprises Compound 1 and a polymer.

In other embodiments, the pharmaceutical composition comprises a solid dispersion a filler, a disintegrant, a surfactant, a binder, a glidant, and a lubricant, wherein the solid dispersion comprises from about 45 wt% to about 65 wt% (e.g., about 50 wt%) of Compound 1 by weight of the dispersion and a polymer.

In some embodiments, the pharmaceutical composition comprises a solid dispersion a filler, a disintegrant, a surfactant, a binder, a glidant, and a lubricant, wherein the solid dispersion comprises from about 75 wt% to about 95 wt% (e.g., about 80 wt%) of Compound 1 by weight of the dispersion and a polymer.

Suitable solid dispersions of Compound 1, i.e., N-[2,4-bis(1,1-dimethylethyl)-5-hydroxyphenyl]-1,4-dihydro-4-oxoquinoline-3-carboxamide, include, without limitation, those dispersions described in PCT publication no. WO 2007/079139, which is hereby incorporated by reference in its entirety.

In one embodiment, the pharmaceutical composition of the present invention comprises a solid dispersion of Compound 1. For example, the solid dispersion comprises substantially amorphous Compound 1, where Compound 1 is less than about 15% (e.g., less than about 10% or less than about 5%) crystalline, and at least one polymer. In another example, the solid dispersion comprises amorphous Compound 1, i.e., Compound 1 has about 0% crystallinity. The concentration of Compound 1 in the solid dispersion depends on several factors such as the amount of pharmaceutical composition needed to provide a desired amount of Compound 1 and the desired dissolution profile of the pharmaceutical composition.

Polymers useful in these solid dispersions are inert, pharmaceutically acceptable polymers that are at least partially soluble in water or biological fluids. Polymers can include homopolymers (e.g., polysaccharides) or copolymers (e.g., block copolymers). In one example, the solid dispersion comprises substantially amorphous or amorphous N-[2,4-bis(1,1-dimethylethyl)-5-hydroxyphenyl]-1,4-dihydro-4-oxoquinoline-3-carboxamide and at least one polymer independently selected from hydroxypropylmethylcellulose (HPMC), hydroxypropylmethylcellulose acetate succinate (HPMCAS), vinylpyrrolidone/vinyl acetate copolymer (PVP/VA), polyvinylpyrrolidone (PVP), methacrylic acid/methacrylate copolymers, hydroxypropyl cellulose (HPC), or any combination thereof. In another example, the solid dispersion comprises

substantially amorphous or amorphous N-[2,4-bis(1,1-dimethylethyl)-5-hydroxyphenyl]-1,4-dihydro-4-oxoquinoline-3-carboxamide and HPMCAS or PVP/VA.

In another embodiment, the pharmaceutical composition comprises a solid dispersion that contains substantially amorphous Compound 1 and HPMCAS or PVP/VA, in which the solid dispersion has a mean particle diameter, measured by light scattering (e.g., using a Malvern Mastersizer available from Malvern Instruments in England) of greater than about 5 μm (e.g., greater than about 6 μm, greater than about 7 μm, greater than about 8 μm, or greater than about 10 μm). For example, the pharmaceutical composition comprises a solid dispersion that contains amorphous Compound 1 and HPMCAS or PVP/VA, in which the solid dispersion has a mean particle diameter, measured by light scattering, of greater than about 5 μm (e.g., greater than about 6 μm, greater than about 7 μm, greater than about 8 μm, or greater than about 10 μm). In another example, the pharmaceutical composition comprises a solid dispersion comprising substantially amorphous Compound 1 and HPMCAS, in which the solid dispersion has a mean particle diameter, measured by light scattering, of from about 7 μm to about 25 μm. For instance, the pharmaceutical composition comprises a solid dispersion comprising amorphous Compound 1 and HPMCAS, in which the solid dispersion has a mean particle diameter, measured by light scattering, of from about 7 μm to about 25 μm. In yet another example, the pharmaceutical composition comprises a solid dispersion comprising substantially amorphous Compound 1 and HPMCAS, in which the solid dispersion has a mean particle diameter, measured by light scattering, of from about 10 μm to about 35 μm. For instance, the pharmaceutical composition comprises a solid dispersion comprising amorphous Compound 1 and HPMCAS, in which the solid dispersion has a mean particle diameter, measured by light scattering, of from about 10 μm to about 35 μm. In another example, the pharmaceutical composition comprises a solid dispersion comprising substantially amorphous Compound 1 and HPMCAS or PVP/VA, in which the solid dispersion has a bulk density of about 0.10 g/cc or greater (e.g., 0.15 g/cc or greater, 0.17 g/cc or greater). For instance, the pharmaceutical composition comprising a solid dispersion comprising amorphous Compound 1 and HPMCAS or PVP/VA, in which the solid dispersion has a bulk density of about 0.10 g/cc or greater (e.g., 0.15 g/cc or greater, 0.17 g/cc or greater). In another instance, the pharmaceutical composition comprises a solid dispersion that comprises substantially amorphous Compound 1 and HPMCAS or PVP/VA, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). In still another instance, the pharmaceutical composition comprises a solid dispersion that includes amorphous Compound 1 and HPMCAS or PVP/VA, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). In another example, the pharmaceutical composition comprises a solid dispersion that comprises substantially amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). For instance, the pharmaceutical composition includes a solid dispersion that comprises amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc).

Alternative solid dispersions comprise substantially amorphous or amorphous Compound 1 and HPMCAS or PVP/VA, wherein substantially amorphous Compound 1 or amorphous Compound 1 is present in an amount of at least 20 wt% (e.g., at least 40 wt%, at least 45 wt%, at least 49 wt%, or at least 50 wt%) by weight of the solid dispersion. In some embodiments, the solid dispersion comprises HPMCAS or PVP/VA and from about 20 wt% to about 99 wt% (e.g., from about 40 wt% to about 90 wt%, from about 42 wt% to about 88 wt%, from about 45 wt% to about 85 wt%, or from about 50 wt% to about 80 wt%) of substantially amorphous or amorphous Compound 1 by weight of the solid dispersion. For example, the solid dispersion comprises HPMCAS or PVP/VA and from about 40 wt% to about 60 wt% (e.g., from about 42 wt% to about 57 wt%, from about 45 wt% to about 55 wt%, or from about 47 wt% to about 53 wt%) of substantially amorphous or amorphous Compound 1 by weight of the solid dispersion. In another example, the solid dispersion comprises HPMCAS or PVP/VA and from about 65 wt% to about 95 wt% (e.g., from about 67 wt% to about 92 wt%, from about 70 wt% to about 90 wt%, or from about 72 wt% to about 88 wt%) of substantially amorphous Compound 1 or amorphous Compound 1 by weight of the solid dispersion.

In other embodiments, the solid dispersion comprises 80 wt% or less (e.g., 60 wt% or less, 55 wt% or less, or 50 wt% or less) of polymer (e.g., HPMCAS, PVP/VA, PVP, methacrylic acid/methacrylate copolymer, HPC, or any combination thereof) by weight of solid dispersion. In some instances, the solid dispersion comprises from about 1 wt% to about 80 wt% (e.g., from about 10 wt% to about 60 wt%) of polymer (e.g., HPMCAS, PVP/VA, PVP, methacrylic acid/methacrylate copolymer, HPC, or any combination thereof).

Some solid dispersions comprise from about 40 wt% to about 60 wt% (e.g., from about 42 wt% to about 57 wt%, from about 45 wt% to about 55 wt%, or from about 47 wt% to about 53 wt%) of substantially amorphous Compound 1 by weight of the solid dispersion and from about 60 wt% to about 40 wt% of polymer (e.g., HPMCAS, PVP/VA, PVP, methacrylic acid/methacrylate copolymer, HPC, or any combination thereof). Alternative solid dispersions comprise from about 40 wt% to about 60 wt% (e.g., from about 42 wt% to about 57 wt%, from about 45 wt% to about 55 wt%, or from about 47 wt% to about 53 wt%) of amorphous Compound 1 by weight of the solid dispersion and from about 60 wt% to about 40 wt% of polymer (e.g., HPMCAS, PVP/VA, PVP, methacrylic acid/methacrylate copolymer, HPC, or any combination thereof).

Other solid dispersions comprise from about 65 wt% to about 95 wt% (e.g., from about 67 wt% to about 92 wt%, from about 70 wt% to about 90 wt%, or from about 72 wt% to about 88 wt%) of substantially amorphous Compound 1 by weight of the solid dispersion and from about 45 wt% to about 5 wt% of polymer (e.g., HPMCAS, PVP/VA, PVP, methacrylic acid/methacrylate copolymer, HPC, or any combination thereof). For instance, the solid dispersion comprises from about 65 wt% to about 95 wt% (e.g., from about 67 wt% to about 92 wt%, from about 70 wt% to about 90 wt%, or from about 72 wt% to about 88 wt%) of amorphous Compound 1 by weight of the solid dispersion and from about 45 wt% to about 5 wt% of polymer (e.g., HPMCAS, PVP/VA, PVP, methacrylic acid/methacrylate copolymer, HPC, or any combination thereof).

Solid dispersions useful in embodiments of the present invention can optionally comprise a surfactant. Suitable surfactants include sodium lauryl sulfate (SLS), sodium stearyl fumarate (SSF), polyoxyethylene 20 sorbitan mono-oleate (e.g., Tween™), any combination thereof, or the like. In one example, the solid dispersion comprises less than 5 wt% (less than 3.0 wt%, less than 1.5 wt%, or less than 1.0 wt%) of surfactant by weight of solid dispersion. In another example, the solid dispersion comprises from about 0.30 wt% to about 0.80 wt% (e.g., from about 0.35 wt% to about 0.70 wt%, from about 0.40 wt% to about 0.60 wt%, or from about 0.45 wt% to about 0.55 wt%) of surfactant by weight of solid dispersion.

In alternative embodiments, the solid dispersion comprises from about 45 wt% to about 85 wt% of substantially amorphous or amorphous Compound 1, from about 0.45 wt% to about 0.55 wt% of SLS, and from about 14.45 wt% to about 55.55 wt% of HPMCAS or PVP/VA by weight of the solid dispersion. One exemplary solid dispersion contains about 50 wt% of substantially amorphous or amorphous Compound 1, about 49.5 wt% of HPMCAS or PVP/VA, and about 0.5 wt% of SLS, by weight of the solid dispersion. Another exemplary solid dispersion contains about 80 wt% of substantially amorphous or amorphous Compound 1, about 19.5 wt% of HPMCAS or PVP/VA, and about 0.5 wt% of SLS.

In alternative embodiments, the solid dispersion comprises from about 45 wt% to about 85 wt% of substantially amorphous or amorphous Compound 1, from about 0.45 wt% to about 0.55 wt% of SLS, and from about 14.45 wt% to about 55.55 wt% of HPMCAS by weight of the solid dispersion. One exemplary solid dispersion contains about 50 wt% of substantially amorphous or amorphous Compound 1, about 49.5 wt% of HPMCAS, and about 0.5 wt% of SLS, by weight of the solid dispersion. Another exemplary solid dispersion contains about 80 wt% of substantially amorphous or amorphous Compound 1, about 19.5 wt% of HPMCAS or PVP/VA, and about 0.5 wt% of SLS.

In addition to the solid dispersion of Compound 1, pharmaceutical compositions of the present invention also comprise one or more excipients such as fillers, disintegrants, surfactants, binders, glidants, lubricants, colorants, or fragrances.

Fillers suitable for the present invention are compatible with the ingredients of the pharmaceutical composition, i.e., they do not substantially reduce the solubility, the hardness, the chemical stability, the physical stability, or the biological activity of the pharmaceutical composition. Exemplary fillers include lactose, sorbitol, celluloses, calcium phosphates, starches, sugars (e.g., mannitol, sucrose, or the like), or any combination thereof. In one embodiment, the pharmaceutical composition comprises at least one filler in an amount of at least about 10 wt% (e.g., at least about 20 wt%, at least about 25 wt%, or at least about 27 wt%) by weight of the composition. For example, the pharmaceutical composition comprises from about 10 wt% to about 60 wt% (e.g., from about 20 wt% to about 55 wt%, from about 25 wt% to about 50 wt%, or from about 27 wt% to about 45 wt%) of filler, by weight of the composition. In another example, the pharmaceutical composition comprises at least about 20 wt% (e.g., at least 25 wt% or at least 27 wt%) of lactose, by weight of the composition. In yet another example, the pharmaceutical composition comprises from about 20 wt% to about 60 wt% (e.g., from about 25 wt% to about 55 wt% or from about 27 wt% to about 45 wt%) of lactose, by weight of the composition.

Disintegrants suitable for the present invention enhance the dispersal of the pharmaceutical composition and are compatible with the ingredients of the pharmaceutical composition, i.e., they do not substantially reduce the chemical stability, the physical stability, the hardness, or the biological activity of the pharmaceutical composition. Exemplary disintegrants include sodium croscarmellose, sodium starch glycolate, or a combination thereof. In one embodiment, the pharmaceutical composition comprises disintegrant in an amount of about 10 wt% or less (e.g., about 7 wt% or less, about 6 wt% or less, or about 5 wt% or less) by weight of the composition. For example, the pharmaceutical composition comprises from about 1 wt% to about 10 wt% (e.g., from about 1.5 wt% to about 7.5 wt% or from about 2.5 wt% to about 6 wt%) of disintegrant, by weight of the composition. In another example, the pharmaceutical composition comprises about 10 wt% or less (e.g., 7 wt% or less, 6 wt% or less, or 5 wt% or less) of sodium croscarmellose, by weight of the composition. In yet another example, the pharmaceutical composition comprises from about 1 wt% to about 10 wt% (e.g., from about 1.5 wt% to about 7.5 wt% or from about 2.5 wt% to about 6 wt%) of sodium croscarmellose, by weight of the composition. In some examples, the pharmaceutical composition comprises from about 0.1% to about 10 wt% (e.g., from about 0.5 wt% to about 7.5 wt% or from about 1.5 wt% to about 6 wt%) of disintegrant, by weight of the composition. In still other examples, the pharmaceutical composition comprises from about 0.5% to about 10 wt% (e.g., from about 1.5 wt% to about 7.5 wt% or from about 2.5 wt% to about 6 wt%) of disintegrant, by weight of the composition.

Surfactants suitable for the present invention enhance the solubility of the pharmaceutical composition and are compatible with the ingredients of the pharmaceutical composition, i.e., they do not substantially reduce the chemical stability, the physical stability, the hardness, or the biological activity of the pharmaceutical composition. Exemplary surfactants include sodium lauryl sulfate (SLS), sodium stearyl fumarate (SSF), polyoxyethylene 20 sorbitan mono-oleate (e.g., Tween™), any combination thereof, or the like. In one embodiment, the pharmaceutical composition comprises a surfactant in an amount of about 10 wt% or less (e.g., about 5 wt% or less, about 2 wt% or less, about 1 wt% or less, about 0.8 wt% or less, or about 0.6 wt% or less) by weight of the composition. For example, the pharmaceutical composition includes from about 10 wt% to about 0.1 wt% (e.g., from about 5 wt% to about 0.2 wt% or from about 2 wt% to about 0.3 wt%) of surfactant, by weight of the composition. In another example, the pharmaceutical composition comprises 10 wt% or less (e.g., about 5 wt% or less, about 2 wt% or less, about 1 wt% or less, about 0.8 wt% or less, or about 0.6 wt% or less) of sodium lauryl sulfate, by weight of the composition. In yet another example, the pharmaceutical composition comprises from about 10 wt% to about 0.1 wt% (e.g., from about 5 wt% to about 0.2 wt% or from about 2 wt% to about 0.3 wt%) of sodium lauryl sulfate, by weight of the composition.

Binders suitable for the present invention enhance the tablet strength of the pharmaceutical composition and are compatible with the ingredients of the pharmaceutical composition, i.e., they do not substantially reduce the chemical stability, the physical stability, or the biological activity of the pharmaceutical composition. Exemplary binders include microcrystalline cellulose, dibasic calcium phosphate, sucrose, corn (maize) starch, modified cellulose (e.g., hydroxymethyl cellulose), or any combination thereof. In one embodiment, the pharmaceutical composition comprises a binder in an amount of at least about 1 wt% (e.g., at least about 10 wt%, at least about 15 wt%, at least about 20 wt%, or at least about 22 wt%) by weight of the composition. For example, the pharmaceutical composition comprises from about 5 wt% to about 50 wt% (e.g., from about 10 wt% to about 45 wt% or from about 20 wt% to about 45 wt%) of binder, by weight of the composition. In another example, the pharmaceutical composition comprises at least about 1 wt% (e.g., at least about 10 wt%, at least about 15 wt%, at least about 20 wt%, or at least about 22 wt%) of microcrystalline cellulose, by weight of the composition. In yet another example, the pharmaceutical composition comprises from about 5 wt% to about 50 wt% (e.g., from about 10 wt% to about 45 wt% or from about 20 wt% to about 45 wt%) of microcrystalline cellulose, by weight of the composition.

Glidants suitable for the present invention enhance the flow properties of the pharmaceutical composition and are compatible with the ingredients of the pharmaceutical composition, i.e., they do not substantially reduce the solubility, the hardness, the chemical stability, the physical stability, or the biological activity of the pharmaceutical composition. Exemplary glidants include colloidal silicon dioxide, talc, or a combination thereof. In one embodiment, the pharmaceutical composition comprises a glidant in an amount of 2 wt% or less (e.g., 1.75 wt%, 1.25 wt% or less, or 1.00 wt% or less) by weight of the composition. For example, the pharmaceutical composition comprises from about 2 wt% to about 0.05 wt% (e.g., from about 1.5 wt% to about 0.07 wt% or from about 1.0 wt% to about 0.09 wt%) of glidant, by weight of the composition. In another example, the pharmaceutical composition comprises 2 wt% or less (e.g., 1.75 wt%, 1.25 wt% or less, or 1.00 wt% or less) of colloidal silicon dioxide, by weight of the composition. In yet another example, the pharmaceutical composition comprises from about 2 wt% to about 0.05 wt% (e.g., from about 1.5 wt% to about 0.07 wt% or from about 1.0 wt% to about 0.09 wt%) of colloidal silicon dioxide, by weight of the composition.

Lubricants suitable for the present invention improve the compression and ejection of compressed pharmaceutical compositions from a die press and are compatible with the ingredients of the pharmaceutical composition, i.e., they do not substantially reduce the solubility, the hardness, or the biological activity of the pharmaceutical composition. Exemplary lubricants include magnesium stearate, stearic acid (stearin), hydrogenated oil, sodium stearyl fumarate, or any combination thereof. In one embodiment, the pharmaceutical composition comprises a lubricant in an amount of 2 wt% or less (e.g., 1.75 wt%, 1.25 wt% or less, or 1.00 wt% or less) by weight of the composition. For example, the pharmaceutical composition comprises from about 2 wt% to about 0.10 wt% (e.g., from about 1.5 wt% to about 0.15 wt% or from about 1.3 wt% to about 0.30 wt%) of lubricant, by weight of the composition. In another example, the pharmaceutical composition comprises 2 wt% or less (e.g., 1.75 wt%, 1.25 wt% or less, or 1.00 wt% or less) of magnesium stearate, by weight of the composition. In yet another example, the pharmaceutical composition comprises from about 2 wt% to about 0.10 wt% (e.g., from about 1.5 wt% to about 0.15 wt% or from about 1.3 wt% to about 0.30 wt%) of magnesium stearate, by weight of the composition.

Pharmaceutical compositions of the present invention can optionally comprise one or more colorants, flavors, and/or fragrances to enhance the visual appeal, taste, and/or scent of the composition. Suitable colorants, flavors, or fragrances are compatible with the ingredients of the pharmaceutical composition, i.e., they do not substantially reduce the solubility, the chemical stability, the physical stability, the hardness, or the biological activity of the pharmaceutical composition. In one embodiment, the pharmaceutical composition comprises a colorant, a flavor, and/or a fragrance. For example, the pharmaceutical composition comprises less than about 1 wt% (e.g., less than about 0.75 wt% or less than about 0.5 wt%) of each optionally ingredient, i.e., colorant, flavor and/or fragrance, by weight of the composition. In another example, the pharmaceutical composition comprises less than about 1 wt% (e.g., less than about 0.75 wt% or less than about 0.5 wt%) of a colorant. In still another example, the pharmaceutical composition comprises less than

about 1 wt% (e.g., less than about 0.75 wt% or less than about 0.5 wt%) of a blue colorant (e.g., FD&C Blue #1 and/or FD&C Blue #2 Aluminum Lake, commercially available from Colorcon, Inc. of West Point, PA.)

In some embodiments, the pharmaceutical composition can be made into tablets and the tablets can be coated with a colorant and optionally labeled with a logo, other image and/or text using a suitable ink. In still other embodiments, the pharmaceutical composition can be made into tablets and the tablets can be coated with a colorant, waxed, and optionally labeled with a logo, other image and/or text using a suitable ink. Suitable colorants and inks are compatible with the ingredients of the pharmaceutical composition, i.e., they do not substantially reduce the solubility, the chemical stability, the physical stability, the hardness, or the biological activity of the pharmaceutical composition. The suitable colorants and inks can be any color and are water based or solvent based. In one embodiment, tablets made from the pharmaceutical composition are coated with a colorant and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a blue colorant (e.g., OPADRY® II, commercially available from Colorcon, Inc. of West Point, PA.). The colored tablets can be labeled with a logo and text indicating the strength of the active ingredient in the tablet using a black ink (e.g., Opacode® WB, commercially available from Colorcon, Inc. of West Point, PA.). In another embodiment, tablets made from the pharmaceutical composition are coated with a colorant, waxed, and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be waxed with Carnauba wax powder weighed out in the amount of about 0.01% w/w of the starting tablet core weight. The waxed tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a blue colorant (e.g., OPADRY® II, commercially available from Colorcon, Inc. of West Point, PA.). The colored tablets can be waxed with Carnauba wax powder weighed out in the amount of about 0.01% w/w of the starting tablet core weight. The waxed tablets can be labeled with a logo and text indicating the strength of the active ingredient in the tablet using a black ink (e.g., Opacode® S-1-17823 - a solvent based ink, commercially available from Colorcon, Inc. of West Point, PA.).

One exemplary pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 40 wt% to about 60 wt% of substantially amorphous Compound 1, by weight of the dispersion, and from about 60 wt% to about 40 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant. Or, the pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 40 wt% to about 60 wt% of amorphous Compound 1, by weight of the dispersion, and from about 60 wt% to about 40 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant.

Another exemplary pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 70 wt% to about 90 wt% of substantially amorphous Compound 1, by weight of the dispersion, and from about 30 wt% to about 10 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant. Or, the pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 70 wt% to about 90 wt% of amorphous Compound 1, by weight of the dispersion, and from about 30 wt% to about 10 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant.

One pharmaceutical composition of the present invention comprises about 15 wt% of a solid dispersion by weight of the composition, wherein the dispersion comprises about 50 wt% of substantially amorphous Compound 1 by weight of the dispersion, about 49.5 wt% of HPMCAS by weight of the dispersion, and about 0.5 wt% SLS by weight of

the dispersion; about 35 wt% of microcrystalline cellulose by weight of the composition; about 43 wt% of lactose by weight of the composition; about 5 wt% of sodium croscarmellose by weight of the composition; about 0.5 wt% of SLS by weight of the composition; about 0.125 wt% of colloidal silicon dioxide by weight of the composition; and about 0.5 wt% of magnesium stearate by weight of the composition. Or, the pharmaceutical composition of the present invention comprises about 15 wt% of a solid dispersion by weight of the composition, wherein the dispersion comprises about 50 wt% of amorphous Compound 1 by weight of the dispersion, about 49.5 wt% of HPMCAS by weight of the dispersion, and about 0.5 wt% SLS by weight of the dispersion; about 35 wt% of microcrystalline cellulose by weight of the composition; about 43 wt% of lactose by weight of the composition; about 5 wt% of sodium croscarmellose by weight of the composition; about 0.5 wt% of SLS by weight of the composition; about 0.125 wt% of colloidal silicon dioxide by weight of the composition; and about 0.5 wt% of magnesium stearate by weight of the composition.

Another pharmaceutical composition of the present invention comprises about 31 wt% of a solid dispersion by weight of the composition, wherein the dispersion comprises about 50 wt% of substantially amorphous Compound 1 by weight of the dispersion, about 49.5 wt% of HPMCAS by weight of the dispersion, and about 0.5 wt% SLS by weight of the dispersion; about 25 wt% of microcrystalline cellulose by weight of the composition; about 38 wt% of lactose by weight of the composition; about 5 wt% of sodium croscarmellose by weight of the composition; about 0.5 wt% of SLS by weight of the composition; about 0.125 wt% of colloidal silicon dioxide by weight of the composition; and about 0.5 wt% of magnesium stearate by weight of the composition. Or, the pharmaceutical composition of the present invention comprises about 31 wt% of a solid dispersion by weight of the composition, wherein the dispersion comprises about 50 wt% of amorphous Compound 1 by weight of the dispersion, about 49.5 wt% of HPMCAS by weight of the dispersion, and about 0.5 wt% SLS by weight of the dispersion; about 25 wt% of microcrystalline cellulose by weight of the composition; about 38 wt% of lactose by weight of the composition; about 5 wt% of sodium croscarmellose by weight of the composition; about 0.5 wt% of SLS by weight of the composition; about 0.125 wt% of colloidal silicon dioxide by weight of the composition; and about 0.5 wt% of magnesium stearate by weight of the composition.

Another pharmaceutical composition of the present invention comprises about 40 wt% of a solid dispersion by weight of the composition, wherein the dispersion comprises about 80 wt% of substantially amorphous Compound 1 by weight of the dispersion, about 19.5 wt% of PVP/VA by weight of the dispersion, and about 0.5 wt% SLS by weight of the dispersion; about 27 wt% of microcrystalline cellulose by weight of the composition; about 27 wt% of lactose by weight of the composition; about 3 wt% of sodium croscarmellose by weight of the composition; about 0.5 wt% of SLS by weight of the composition; about 1 wt% of colloidal silicon dioxide by weight of the composition; about 1 wt% of magnesium stearate by weight of the composition, and about 0.4 wt% of colorant by weight of the composition. Or, the pharmaceutical composition of the present invention comprises about 40 wt% of a solid dispersion by weight of the composition, wherein the dispersion comprises about 80 wt% of amorphous Compound 1 by weight of the dispersion, about 19.5 wt% of PVP/VA by weight of the dispersion, and about 0.5 wt% SLS by weight of the dispersion; about 27 wt% of microcrystalline cellulose by weight of the composition; about 27 wt% of lactose by weight of the composition; about 3 wt% of sodium croscarmellose by weight of the composition; about 0.5 wt% of SLS by weight of the composition; about 1 wt% of colloidal silicon dioxide by weight of the composition; about 1 wt% of magnesium stearate by weight of the composition, and about 0.4 wt% of colorant by weight of the composition.

Another pharmaceutical composition of the present invention comprises about 40 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 27 wt% of microcrystalline cellulose by weight of the composition; about 27 wt% of lactose by weight of the composition; about 3 wt% of sodium croscarmellose by weight of the composition; about 0.5 wt% of SLS by weight of the composition; about 1 wt% of colloidal silicon dioxide by weight of the composition; about 1 wt% of magnesium stearate by weight of the composition, and about 0.4 wt% of colorant by weight of the composition. Or, the pharmaceutical composition of the present invention comprises about 40 wt% of a solid dispersion by weight of the composition, wherein the dispersion comprises about 80 wt% of amorphous Compound 1 by weight of the dispersion, about 19.5 wt% of HPMCAS by weight of the dispersion, and about 0.5 wt% SLS by weight of the dispersion; about 27 wt% of microcrystalline cellulose by weight of the composition; about 27 wt% of lactose by weight of the composition; about 3 wt% of sodium croscarmellose by weight of the composition; about 0.5 wt% of SLS by weight of the composition; about 1 wt% of colloidal silicon dioxide by weight of the composition; about 1 wt% of magnesium stearate by weight of the composition, and about 0.4 wt% of colorant by weight of the composition.

In still another pharmaceutical composition of the present invention comprises about 34.5 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30 wt% of lactose by weight of the composition; about 3 wt% of sodium croscarmellose by weight of the composition; about 0.5 wt% of SLS by weight of the composition; about 1 wt% of colloidal silicon dioxide by weight of the composition; about 1 wt% of magnesium stearate by weight of the composition.

In yet a further pharmaceutical composition of the present invention, a caplet shaped pharmaceutical tablet composition having a hardness of 9.5 Kp ± 15 percent comprises about 34 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In certain embodiments, the caplet shaped pharmaceutical tablet contains 150 mg of Compound 1. In certain embodiments, the caplet shaped pharmaceutical tablet contains 100 mg of Compound 1.

In still another pharmaceutical composition of the present invention, a caplet shaped pharmaceutical tablet composition having an initial hardness of 11 Kp ± 20 percent comprises about 40 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In certain embodiments, the caplet shaped pharmaceutical tablet contains 150 mg of Compound 1.

In still another pharmaceutical composition of the present invention, a caplet shaped pharmaceutical tablet composition having an initial hardness of 11 Kp ± 20 percent comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In some aspects, the caplet shaped pharmaceutical tablet composition contains 100 mg of Compound 1. In other aspects, the caplet shaped pharmaceutical tablet composition includes a colorant coating and a printed logo or text. In some embodiments of this aspect, the caplet shaped pharmaceutical tablet composition includes a blue OPADRY® II coating and a water or solvent based ink logo or text. In certain embodiments, the caplet shaped pharmaceutical tablet contains 150 mg of Compound 1.

In another pharmaceutical composition of the present invention, a caplet shaped pharmaceutical tablet composition having an initial hardness of between about 6 and 16 Kp comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some aspects, the caplet shaped pharmaceutical tablet composition contains 100 mg of Compound 1. In some further aspects, the caplet shaped pharmaceutical tablet composition comprises a colorant coated, a wax coating, and a printed logo or text. In some embodiments of this aspect, the caplet shaped pharmaceutical tablet includes a blue OPADRY® II coating and a water or solvent based ink logo or text. In some instances, the colorant coating is blue OPADRY® II. In some instances, the wax coating comprises Carnauba wax. In certain aspects, the ink for the printed logo or text is a solvent based ink. In some aspects, the caplet shaped pharmaceutical tablet composition contains 150 mg of Compound 1.

In still another pharmaceutical composition of the present invention, a pharmaceutical tablet composition having an initial hardness of between about 9 and 21 Kp comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some embodiments, the caplet shaped pharmaceutical tablet composition contains 150 mg of Compound 1. In some aspects, the caplet shaped pharmaceutical tablet composition further comprises a colorant coated, a wax coating, and a printed logo or text. In some instances, the tablet includes a blue OPADRY® II coating and a water or solvent based ink logo or text. In still other instances, the wax coating comprises Carnauba wax. In some embodiments, the ink for the printed logo or text is a solvent based ink. In some aspects, the caplet shaped pharmaceutical tablet composition contains 100 mg of Compound 1.

In yet a further pharmaceutical composition of the present invention, a pharmaceutical composition comprises

about 34 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In certain embodiments, the pharmaceutical composition contains 150 mg of Compound 1. In other embodiments, the pharmaceutical composition contains 100 mg of Compound 1.

In still another pharmaceutical composition of the present invention, a pharmaceutical composition comprises about 40 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In certain embodiments, the pharmaceutical composition contains 150 mg of Compound 1. In other embodiments, the pharmaceutical composition contains 100 mg of Compound 1.

In still another pharmaceutical composition of the present invention, a pharmaceutical composition comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 1 wt% of colloidal silicon dioxide by weight of the composition; and about 1 wt% of magnesium stearate by weight of the composition. In some aspects, the pharmaceutical composition contains 100 mg of Compound 1. In other embodiments, the pharmaceutical composition contains 150 mg of Compound 1. In other aspects, the pharmaceutical composition is formed as a tablet composition that includes a colorant coating and a printed logo or text. In some embodiments of this aspect, the pharmaceutical tablet composition includes a blue OPADRY® II coating and a water or solvent based ink logo or text.

In another pharmaceutical composition of the present invention, a pharmaceutical composition comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some aspects, the pharmaceutical tablet contains 100 mg of Compound 1. In other embodiments, the pharmaceutical composition contains 150 mg of Compound 1. In some further aspects, the pharmaceutical composition is formed as a tablet and comprises a colorant coated, a wax coating, and a printed logo or text. In some embodiments of this aspect, the pharmaceutical tablet includes a blue OPADRY® II coating and a water or solvent based ink logo or text. In some instances, the colorant coating is blue OPADRY® II. In some instances, the wax coating comprises Carnauba wax. In certain aspects, the ink for the printed logo or text is a solvent based ink.

It is also noted that pharmaceutical compositions of the present invention can be processed into a tablet form, capsule form, or suspension that is suited for oral administration or can be reconstituted in an aqueous solvent (e.g., DI water or saline) for oral, IV, or inhalation (e.g., nebulizer) administration.

Another aspect of the present invention provides a pharmaceutical composition consisting of a tablet that includes a CF potentiator API (e.g., a solid dispersion of N-[2,4-bis(1,1-dimethylethyl)-5-hydroxyphenyl]-1,4-dihydro-4-oxoquinoline-3-carboxamide) and other excipients (e.g., a filler, a disintegrant, a surfactant, a binder, a glidant, a colorant, a lubricant, or any combination thereof), each of which is described above and in the Examples below, wherein the tablet has a dissolution of at least about 50% (e.g., at least about 60%, at least about 70%, at least about 80%, at least about 90%, or at least about 99%) in about 30 minutes. In one example, the pharmaceutical composition consists of a tablet that includes a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a dissolution of from about 50% to about 100% (e.g., from about 55% to about 95% or from about 60% to about 90%) in about 30 minutes. In another example, the pharmaceutical composition consists of a tablet that comprises a solid dispersion comprising substantially amorphous or amorphous Compound 1 and HPMCAS or PVP/VA; and, a filler, a disintegrant, a surfactant, a binder, a glidant, and a lubricant, wherein the tablet has a dissolution of at least about 50% (e.g., at least about 60%, at least about 70%, at least about 80%, at least about 90%, or at least about 99%) in about 30 minutes. In still another example, the pharmaceutical

composition consists of a tablet that comprises a solid dispersion comprising substantially amorphous or amorphous Compound 1 and HPMCAS or PVP/VA; and, a filler, a disintegrant, a surfactant, a binder, a glidant, and a lubricant, wherein the tablet has a dissolution of from about 50% to about 100% (e.g., from about 55% to about 95% or from about 60% to about 90%) in about 30 minutes.

In one embodiment, the tablet comprises a solid dispersion comprising at least about 25 mg (e.g., at least about 30 mg, at least about 40 mg, or at least about 50 mg) of substantially amorphous or amorphous Compound 1; and PVP/VA and SLS. In another embodiment, the tablet comprises a solid dispersion comprising at least about 25 mg (e.g., at least about 30 mg, at least about 40 mg, at least about 50 mg, at least about 100 mg, or at least 150 mg) of substantially amorphous or amorphous Compound 1; and HPMCAS and SLS.

Dissolution can be measured with a standard USP Type II apparatus that employs a dissolution media of 0.6% sodium lauryl sulfate dissolved in 900 mL of DI water, stirring at about 50-75 rpm at a temperature of about 37 °C. A single experimental tablet is tested in each test vessel of the apparatus. Dissolution can also be measured with a standard USP Type II apparatus that employs a dissolution media of 0.7% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus. Dissolution can also be measured with a standard USP Type II apparatus that employs a dissolution media of 0.5% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus.

Another aspect of the present invention provides a pharmaceutical composition consisting of a tablet that comprises a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a hardness of at least about 5 Kp. In one example, the pharmaceutical composition consists of a tablet that comprises a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a hardness of at least about 5 Kp (e.g., at least about 5.5, at least about 6 Kp, or at least about 7 Kp).

## III. METHOD OF PRODUCING A PHARMACEUTICAL COMPOSITION

Another aspect of the present invention provides a method of producing a pharmaceutical composition comprising providing an admixture of a solid dispersion of substantially amorphous or amorphous N-[2,4-Bis(1,1-dimethylethyl)-5-hydroxyphenyl]-1,4-dihydro-4-oxoquinoline-3-carboxamide, a binder, a glidant, a surfactant, a lubricant, a disintegrant, and a filler, and compressing the admixture into a tablet having a dissolution of at least about 50% in about 30 minutes.

Each of the ingredients of this admixture is described above and in the Examples below. Furthermore, the admixture can comprise optional additives such as one or more colorants, one or more flavors, and/or one or more fragrances as described above and in the Examples below. And, the relative concentrations (e.g., wt%) of each of these ingredients (and any optional additives) in the admixture is also presented above and in the Examples below. The ingredients constituting the admixture can be provided sequentially or in any combination of additions; and, the ingredients or combination of ingredients can be provided in any order. In one embodiment the lubricant is the last component added to the admixture.

In one embodiment, the admixture comprises a solid dispersion of substantially amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less)). For instance, the admixture comprises a solid dispersion of amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less)).

In another embodiment, the admixture comprises a solid dispersion of substantially amorphous Compound 1, a binder, a glidant, a surfactant, a lubricant, a disintegrant, and a filler, wherein each of these ingredients is substantially free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient. For instance, the admixture comprises a solid dispersion of amorphous Compound 1, a binder, a glidant, a surfactant, a lubricant, a disintegrant, and a filler, wherein each of these ingredients is substantially free of water. To wit, each of the ingredients comprises less than 5 wt% (e.g., less than 2 wt%, less than 1 wt%, less than 0.75 wt%, less than 0.5 wt%, or less than 0.25 wt%) of water by weight of the ingredient.

In another embodiment, compressing the admixture into a tablet is accomplished by filling a form (e.g., a mold)

with the admixture and applying pressure to admixture. This can be accomplished using a die press or other similar apparatus. It is also noted that the application of pressure to the admixture in the form can be repeated using the same pressure during each compression or using different pressures during the compressions. In another example, the admixture is compressed using a die press that applies sufficient pressure to form a tablet having a dissolution of about 50% or more at about 30 minutes (e.g., about 55% or more at about 30 minutes or about 60% or more at about 30 minutes). For instance, the admixture is compressed using a die press to produce a tablet hardness of at least about 5 Kp (at least about 5.5 Kp, at least about 6 Kp, at least about 7 Kp, at least about 11 Kp, or at least 21Kp). In some instances, the admixture is compressed to produce a tablet hardness of between about 6 and 21 Kp.

In some embodiments, tablets comprising a pharmaceutical composition as described herein can be coated with about 3.0 wt% of a film coating comprising a colorant by weight of the tablet. In certain instances, the colorant suspension or solution used to coat the tablets comprises about 20%w/w of solids by weight of the colorant suspension or solution. In still further instances, the coated tablets can be labeled with a logo, other image or text.

In another embodiment, the method of producing a pharmaceutical composition comprises providing an admixture of a solid dispersion of substantially amorphous Compound 1, a binder, a glidant, a surfactant, a lubricant, a disintegrant, and a filler; mixing the admixture until the admixture is substantially homogenous, and compressing the admixture into a tablet as described above or in the Examples below. Or, the method of producing a pharmaceutical composition comprises providing an admixture of a solid dispersion of amorphous Compound 1, a binder, a glidant, a surfactant, a lubricant, a disintegrant, and a filler; mixing the admixture until the admixture is substantially homogenous, and compressing the admixture into a tablet as described above or in the Examples below. For example, the admixture is mixed by stirring, blending, shaking, or the like using hand mixing, a mixer, a blender, any combination thereof, or the like. When ingredients or combinations of ingredients are added sequentially, mixing can occur between successive additions, continuously throughout the ingredient addition, after the addition of all of the ingredients or combinations of ingredients, or any combination thereof. The admixture is mixed until it has a substantially homogenous composition.

## IV. ADMINISTRATION OF A PHARMACEUTICAL FORMULATION

In another aspect, the invention also provides a method of treating or lessening the severity of a disease in a patient comprising administering to said patient one of the compositions as defined herein, and said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, Gorham's Syndrome, chloride channelopathies such as myotonia congenita (Thomson and Becker forms), Bartter's syndrome type III, Dent's disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 25 mg of substantially amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 50 mg of substantially amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 75 mg of substantially

amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 100mg of substantially amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 250 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 25 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 50 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 75 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 250 mg of substantially amorphous or amorphous Compound 1.

Another aspect of the present invention provides a method of administering a pharmaceutical composition by orally administering to a patient once every 12 hours. The composition comprises a solid dispersion of substantially amorphous or amorphous Compound 1, in which the solid dispersion comprises at least about 25 mg of substantially amorphous or amorphous Compound 1.

Another aspect of the present invention provides a method of administering a pharmaceutical composition by orally administering to a patient once every 12 hours. The composition comprises a solid dispersion of substantially amorphous or amorphous Compound 1, in which the solid dispersion comprises at least about 50 mg of substantially amorphous or amorphous Compound 1.

Another aspect of the present invention provides a method of administering a pharmaceutical composition by orally administering to a patient once every 12 hours. The composition comprising a solid dispersion of substantially amorphous or amorphous Compound 1, in which the solid dispersion comprises at least about 75 mg of substantially amorphous or amorphous Compound 1.

Another aspect of the present invention provides a method of administering a pharmaceutical composition by orally administering to a patient once every 12 hours day. The composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient once every 12 hours. The composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by

orally administering to a patient once every 12 hours. The composition comprising a solid dispersion of substantially amorphous or amorphous Compound 1, in which the solid dispersion comprises at least about 250 mg of substantially amorphous or amorphous Compound 1.

In still other aspects of the present invention, a pharmaceutical composition as described herein is orally administered to a patient once every 24 hours.

Another aspect of the present invention provides a method of administering a pharmaceutical composition by orally administering to a patient once per day the composition comprising a solid dispersion of substantially amorphous or amorphous Compound 1, in which the solid dispersion comprises at least about 25 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 50 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 75 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 250 mg of substantially amorphous or amorphous Compound 1.

Another aspect of the present invention provides a method of administering a pharmaceutical composition comprising orally administering to a patient at least once per day at least one tablet comprising a pharmaceutical composition containing a solid dispersion of substantially amorphous Compound 1, a filler, a binder, a glidant, a disintegrant, a surfactant, and a lubricant, each of which is described above and in the Examples below, wherein the solid dispersion comprises at least 25 mg (e.g., at least 35 mg, at least 40 mg, or at least 45 mg) of substantially amorphous Compound 1.

In some embodiments, the present invention provides a method of administering a pharmaceutical composition comprising orally administering to a patient at least one tablet comprising:

a. a solid dispersion comprising about 25 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 50 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition

comprising orally administering to a patient at least one tablet comprising:

a. a solid dispersion comprising about 75 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 100 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 150 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 250 mg of substantially amorphous or amorphous Compound 1;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 25 mg of substantially amorphous or amorphous Compound 1 and PVP/VA;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 50 mg of substantially amorphous or amorphous Compound 1 and PVP/VA;

b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 75 mg of substantially amorphous or amorphous Compound 1 and PVP/VA;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 100 mg of substantially amorphous or amorphous Compound 1 and PVP/VA;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 150 mg of substantially amorphous or amorphous Compound 1 and PVP/VA;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 250 mg of substantially amorphous or amorphous Compound 1 and PVP/VA;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 25 mg of substantially amorphous or amorphous Compound 1 and HPMCAS;
b. a filler;
c. a disintegrant;
d. a surfactant;

e. a binder;
f. a glidant; and
g. a lubricant.

In some embodiments, the present invention provides a method of administering a pharmaceutical composition comprising orally administering to a patient at least one tablet comprising:

a. a solid dispersion comprising about 50 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 75 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 100 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 150 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 250 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides for a method of orally administering the pharmaceutical composition described herein once a day. In other embodiments, the present invention provides for a method of orally administering the pharmaceutical composition described herein twice a day.

Another aspect of the present invention provides a method of administering a pharmaceutical composition by orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion comprises at least about 25 mg of substantially amorphous or amorphous Compound 1. In some embodiments, the tablet is orally administered to the patient once per day. In another method, the administration comprises orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 25 mg of substantially amorphous or amorphous Compound 1. Some tablets useful in this method comprise a solid dispersion containing at least about 50 mg of substantially amorphous or amorphous Compound 1. In another method, the administration includes orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 50 mg of substantially amorphous or amorphous Compound 1. Some tablets useful in this method comprise a solid dispersion containing at least about 75 mg of substantially amorphous or amorphous Compound 1. In another method, the administration includes orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 75 mg of substantially amorphous or amorphous Compound 1. Another aspect of the present invention provides a method of administering a pharmaceutical composition by orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion comprises at least about 100 mg of substantially amorphous or amorphous Compound 1. In some embodiments, the tablet is orally administered to the patient once per day. In another method, the administration comprises orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 100 mg of substantially amorphous or amorphous Compound 1. Other tablets useful in this method comprise a solid dispersion containing at least about 150 mg of substantially amorphous or amorphous Compound 1. In another method, the administration includes orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 150 mg of substantially amorphous or amorphous Compound 1. In another method, the administration includes orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion contains at least about 250 mg of substantially amorphous or amorphous Compound 1. In another method, the administration includes orally administering to a patient once per day at least one tablet comprising a solid dispersion of substantially amorphous or amorphous Compound 1, a filler, a binder, a glidant, a disintegrant, a surfactant, and a lubricant, in which the solid dispersion contains at least about 250 mg of substantially amorphous or amorphous Compound 1. In another method, the administration includes orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 250 mg of substantially amorphous or amorphous Compound 1.

In one embodiment, the method of administering a pharmaceutical composition including orally administering to a patient at least once per day at least one tablet including a pharmaceutical composition containing a solid dispersion of amorphous Compound 1, a filler, a binder, a glidant, a disintegrant, a surfactant, and a lubricant, each of which is described above and in the Examples below, wherein the solid dispersion comprises at least 25 mg (e.g., at least 35 mg, at least 40 mg, or at least 45 mg) of substantially amorphous Compound 1.

In one embodiment, the method of administering a pharmaceutical composition includes orally administering to a patient at least once per day at least one tablet comprising a pharmaceutical composition containing a solid dispersion of substantially amorphous Compound 1, a filler, a binder, a glidant, a disintegrant, a surfactant, and a lubricant, wherein the solid dispersion comprises from about 30 mg to about 300 mg (e.g., from about 40 mg to about 280 mg or from about 45 mg to about 260 mg, or from about 50 mg to about 200 mg) of substantially amorphous Compound 1. Or, the method of administering a pharmaceutical composition includes orally administering to a patient at least once per day at least one tablet comprising a pharmaceutical composition containing a solid dispersion of amorphous Compound 1, a filler, a binder, a glidant, a disintegrant, a surfactant, and a lubricant, wherein the solid dispersion comprises from about 30 mg to about 300 mg (e.g., from about 40 mg to about 280 mg or from about 45 mg to about 260 mg, or from about 50 mg to about 200 mg) of amorphous Compound 1.

In another embodiment, the method of administering a pharmaceutical composition includes orally administering

to a patient once per day at least one tablet comprising a pharmaceutical composition containing a solid dispersion of Compound 1, a filler, a binder, a glidant, a disintegrant, a surfactant, and a lubricant, each of which is described above and in the Examples below, wherein the solid dispersion comprises at least 25 mg (e.g., at least 35 mg, at least 40 mg, at least 45 mg, at least 75 mg, at least about 100 mg, at least about 150 mg, or at least 250 mg,) of substantially amorphous Compound 1 or amorphous Compound 1. For example, the method of administering a pharmaceutical composition includes orally administering to a patient once per day one tablet comprising a pharmaceutical composition containing a solid dispersion of Compound 1, a filler, a binder, a glidant, a disintegrant, a surfactant, and a lubricant, wherein the solid dispersion comprises at least 75 mg (e.g., at least 100 mg, at least 125 mg, at least 140 mg, at least 150 mg, or at least 250 mg) of substantially amorphous Compound 1 or amorphous Compound 1. In another example, the method of administering a pharmaceutical composition includes orally administering to a patient once per day a plurality of tablets (e.g., two tablets, three tablets, four or five tablets), wherein each tablet comprises a pharmaceutical composition comprising a solid dispersion of substantially amorphous Compound 1 or amorphous Compound 1, a filler, a binder, a glidant, a disintegrant, a surfactant, and a lubricant, wherein the solid dispersion comprises at least 25 mg (e.g., at least 35 mg, at least 40 mg, at least 45 mg, at least 75 mg, at least about 150 mg, or at least 250 mg,) of substantially amorphous Compound 1 or amorphous Compound 1.

In another embodiment, the method of administering a pharmaceutical composition includes orally administering to a patient twice per day at least one tablet comprising a pharmaceutical composition containing a solid dispersion of Compound 1, a filler, a binder, a glidant, a disintegrant, a surfactant, and a lubricant, each of which is described above and in the Examples below, wherein the solid dispersion comprises at least 25 mg (e.g., at least 35 mg, at least 40 mg, at least 45 mg, at least 50 mg, at least 75 mg, at least about 150 mg, or at least 250 mg,) of substantially amorphous Compound 1 or amorphous Compound 1. For example, the method of administering a pharmaceutical composition includes orally administering to a patient twice per day one tablet comprising a pharmaceutical composition containing a solid dispersion of Compound 1, a filler, a binder, a glidant, a disintegrant, a surfactant, and a lubricant, wherein the solid dispersion comprises at least 75 mg (e.g., at least 100 mg, at least 125 mg, at least 140 mg, at least 150 mg, or at least 250 mg) of substantially amorphous Compound 1 or amorphous Compound 1. In another example, the method of administering a pharmaceutical composition includes orally administering to a patient twice per day a plurality of tablets (e.g., two tablets, three tablets, four or five tablets), wherein each tablet comprises a pharmaceutical composition comprising a solid dispersion of substantially amorphous Compound 1 or amorphous Compound 1, a filler, a binder, a glidant, a disintegrant, a surfactant, and a lubricant, wherein the solid dispersion comprises at least 25 mg (e.g., at least 35 mg, at least 40 mg, at least 45 mg, at least 50 mg, at least 75 mg, at least about 150 mg, or at least 250 mg,) of substantially amorphous Compound 1 or amorphous Compound 1.

It is noted that the methods of administration of the present invention can optionally include orally administering a beverage (water, milk, or the like), food, and/or additional pharmaceutical compositions including additional APIs. When the method of administration includes orally administering a beverage (water, milk, or the like), food (including a standard high fat high calorie CF meal or snack), and/or additional pharmaceutical compositions including additional APIs, the oral administration of the beverage, food, and/or additional API can occur concurrently with the oral administration of the tablet, prior to the oral administration of the tablet, and/or after the administration of the tablet. For instance, in one example, the method of administering a pharmaceutical composition includes orally administering to a patient at least once per day at least one tablet comprising a pharmaceutical composition containing a solid dispersion of substantially amorphous Compound 1 or amorphous Compound 1, a filler, a binder, a glidant, a disintegrant, a surfactant, a lubricant, and a second API. In another example, the method of administering a pharmaceutical composition includes orally administering to a patient at least once per day at least one tablet comprising a pharmaceutical composition comprising a solid dispersion of substantially amorphous Compound 1 or amorphous Compound 1, a filler, a binder, a glidant, a disintegrant, a surfactant, and a lubricant, wherein the solid dispersion comprises at least 25 mg (e.g., at least 35 mg, at least 45 mg, or at least 50 mg) of substantially amorphous Compound 1 or amorphous Compound 1, and orally administering to a patient at least once per day a second pharmaceutical composition comprising a second API. In still other examples, the method of administering a pharmaceutical composition includes orally administering to a patient every 12 hours at least one tablet comprising a pharmaceutical composition as described herein, in which the tablet is administered about 30 minutes after consuming a high fat, high calorie CF meal or snack.

It will also be appreciated that the compound and pharmaceutically acceptable compositions of the present invention can be employed in combination therapies, that is, the compound and pharmaceutically acceptable compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate

for the disease, or condition, being treated."

In one embodiment, the additional agent is selected from a mucolytic agent, bronchodialator, an anti-biotic, an anti-infective agent, an anti-inflammatory agent, a CFTR modulator other than Compound 1 of the present invention, or a nutritional agent.

In one embodiment, the additional agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations thereof suitable for administration by inhalation, levoflaxacin, including aerosolized formulations thereof, and combinations of two antibiotics, e.g., fosfomycin and tobramycin.

In another embodiment, the additional agent is a mucolyte. Exemplary mucolytes useful herein includes Pulmozyme®.

In another embodiment, the additional agent is a bronchodialator. Exemplary bronchodialtors include albuterol, metaprotenerol sulfate, pirbuterol acetate, salmeterol, or tetrabuline sulfate.

In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3S, 5R)-5-(4-amino-2-oxopyrimidin-1-yl)-3-hydroxyoxolan-2-yl]methoxy-hydroxyphosphoryl] [[[(2R,3S,4R,5R)-5-(2,4-dioxopyrimidin-1-yl)-3, 4-dihydroxyoxolan-2-yl]methoxy-hydroxyphosphoryl]oxy-hydroxyphosphoryl] hydrogen phosphate), or bronchitol (inhaled formulation of mannitol).

In another embodiment, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, or simavastatin.

In another embodiment, the additional agent is a CFTR modulator other than compound 1, i.e., an agent that has the effect of modulating CFTR activity. Exemplary such agents include ataluren ("PTC124®"; 3-[5-(2-fluorophenyl)-1,2,4-oxadiazol-3-yl]benzoic acid), sinapultide, lancovutide, depelestat (a human recombinant neutrophil elastase inhibitor), cobiprostone (7-{(2R, 4aR, 5R, 7aR)-2-[(3S)-1,1-difluoro-3-methylpentyl]-2-hydroxy-6-oxooctahydrocyclopenta[b]pyran-5-yl}heptanoic acid), or (3-(6-(1-(2,2-difluorobenzo[d][1,3]dioxol-5-yl) cyclopropanecarboxamido)-3-methylpyridin-2-yl)benzoic acid. In another embodiment, the additional agent is (3-(6-(1-(2,2-difluorobenzo[d][1,3]dioxol-5-yl) cyclopropanecarboxamido)-3-methylpyridin-2-yl)benzoic acid.

In another embodiment, the additional agent is a nutritional agent. Exemplary such agents include pancrelipase (pancreating enzyme replacement), including Pancrease®, Pancreacarb®, Ultrase®, or Creon®, Liprotomase® (formerly Trizytek®), Aquadeks®, or glutathione inhalation. In one embodiment, the additional nutritional agent is pancrelipase.

## VI. EXAMPLES

In order that the invention described herein may be more fully understood, the following examples are set forth. It should be understood that these examples are for illustrative purposes only and are not to be construed as limiting this invention in any manner.

### A. Manufacture of Tablets

Intermediate A

A solvent system of methylethyl ketone (MEK) and DI water, formulated according to the ratio 90 wt% MEK / 10 wt% DI water, was added to a reactor equipped with a magnetic stirrer and thermal circuit. Into this solvent system, hypromellose acetate succinate polymer (HG grade, commercially available from Biddle Sawyer Corporation in New York, New York or Shin-Etsu Chemical Co. in Tokyo, Japan), sodium lauryl sulfate (SLS), and N-[2,4-bis(1,1-dimethylethyl)-5-hydroxyphenyl]-1,4-dihydro-4-oxoquinoline-3-carboxamide were added according to the ratio 49.5 wt% hypromellose acetate succinate / 0.5 wt% sodium lauryl sulfate (SLS) / 50 wt% N-[2,4-bis(1,1-dimethylethyl)-5-hydroxyphenyl]-1,4-dihydro-4-oxoquinoline-3-carboxamide. The resulting mixture contained 20 wt% dissolved solids. The actual amounts of ingredients and amounts of solvents used to generate this mixture are recited in Table A1, below:

Table A1: Solid Spray Dispersion Ingredients for Intermediate A

| | Units | Batch |
|---|---|---|
| N-[2,4-Bis( 1,1-dimethylethyl)-5-hydroxyphenyl]-1,4-dihydro-4-oxoquinoline-3-carboxamide | Kg | 9.00 |

(continued)

| | Units | Batch |
|---|---|---|
| hypromellose acetate succinate | Kg | 8.91 |
| SLS | Kg | 0.09 |
| **Total Solids** | **Kg** | **18.00** |
| MEK | Kg | 64.80 |
| Water | Kg | 7.20 |
| **Total Solvents** | **Kg** | **72.00** |
| **Total Spray Solution Weight** | **Kg** | **90.00** |

The mixture was mixed at room temperature until it was substantially homogenous and all components were substantially dissolved.

A spray drier, Niro Mobile Minor Spray Dryer with extended chamber, fitted with a 1.3 mm two-fluid atomizer situated approximately 5 cm from the top of the spray drying vessel was used in accordance with the spray dry parameters in Table A2.

Table A2: Dry spray process parameters used to generate Intermediate A.

| **Parameter** | **Value** |
|---|---|
| Atomization Flow Rate | 10.5 kg/hr |
| Feed Flow Rate | 7 kg/hr |
| Inlet Temperature | ~105°C |
| Outlet Temperature | 40 °C ± 5 °C |
| Vacuum Dryer Temperature | 55 °C |
| Vacuum Drying Time | 24 hours |

An inertial cyclone separated the product from the process gas and solvent vapors, and a filter bag collected the fine particles not separated by the cyclone. The resultant product was transferred to a vacuum tray dryer for drying to reduce residual solvents to a level of less than about 5000 ppm and to generate dry Intermediate A.

Intermediate B

A solvent system of MEK, DI water, and acetone, formulated according to the ratio 65 wt% MEK / 9 wt% DI water / 26 wt% acetone, was heated to a temperature of 20 - 30 °C in a reactor equipped with a magnetic stirrer and thermal circuit. Into this solvent system, a copolymer of vinylpyrrolidone and vinylacetatepolyvinylpyrrolidone (PVP VA-64 commercially available from Shanghai Lite Chemical Technology Co., Ltd. Shanghai, China), SLS, and N-[2,4-Bis(1,1-dimethylethyl)-5-hydroxyphenyl]-1,4-dihydro-4-oxoquinoline-3-carboxamide were added according to the ratio 19.5 wt% PVPVA-64 / 0.5 wt% sodium lauryl sulfate / 80 wt% N-[2,4-Bis(1,1-dimethylethyl)-5-hydroxyphenyl]-1,4-dihydro-4-oxoquinoline-3-carboxamide. The resulting mixture contained 11.5 wt% solids. The actual amounts of ingredients and solvents used to generate this mixture are recited in Table B1, below:

Table B1: Solid Spray Dispersion Ingredients for Intermediate B

| | Units | Batch 1 |
|---|---|---|
| N-[2,4-Bis(1,1-dimethylethyl)-5-hydroxyphenyl]-1,4-dihydro-4-oxoquinoline-3-carboxamide | Kg | 24.00 |
| PVPVA-64 | Kg | 5.850 |
| SLS | Kg | 0.1500 |
| **Total Solids** | **Kg** | **30.00** |

(continued)

| | Units | Batch 1 |
|---|---|---|
| MEK | Kg | 150.1 |
| Water | Kg | 20.78 |
| Acetone | Kg | 60.03 |
| **Total Solvents** | **Kg** | **230.9** |
| **Total Spray Solution Weight** | **Kg** | **260.9** |

The mixture was maintained at a temperature of 20 - 30 °C and mixed until it was substantially homogenous and all components were substantially dissolved.

A spray drier, Niro Production Minor Spray Dryer, fitted with pressure nozzles (Spray Systems Maximum Passage series SK-MFP having orifice size 72), was used under normal spray drying mode, following the dry spray process parameters recited in Table B2, below. The spray nozzle was situated approximately 5 cm from the top of the spray drying vessel.

Table B2: Dry spray process parameters used to generate Intermediate B.

| **Parameter** | **Value** |
|---|---|
| Feed Pressure | 30 - 100 bar |
| Feed Flow Rate | 15 - 25 Kg/hr |
| Inlet Temperature | 85 - 125 °C |
| Outlet Temperature | 45 - 75 °C |
| Vacuum Dryer Temperature | 55°C ± 5 °C |
| Vacuum Drying Time | 24 hours |

A high efficiency cyclone separated the wet product from the spray gas and solvent vapors. The wet product was transferred to a tray vacuum dryer for drying to reduce residual solvents to a level of less than about 5000 ppm and to generate dry Intermediate B.

Intermediate C:

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt% hypromellose acetate succinate / 0.5 wt% SLS / 80 wt% N-[2,4-Bis(1,1-dimethylethyl)-5-hydroxyphenyl]-1,4-dihydro-4-oxoquinoline-3-carboxamide. The resulting mixture contained 12.5 wt% solids. The actual amounts of ingredients and solvents used to generate this mixture are recited in Table C1, below:

Table C1: Solid Spray Dispersion Ingredients for Intermediate C.

| | Units | Batch |
|---|---|---|
| N-[2,4-Bis(1,1-dimethylethyl)-5-hydroxyphenyl]-1,4-dihydro-4-oxoquinoline-3-carboxamide | Kg | 24.00 |
| HPMCAS | Kg | 5.850 |
| SLS | Kg | 0.1500 |
| **Total Solids** | **Kg** | **30.00** |
| MEK | Kg | 189.0 |
| Water | Kg | 21.00 |

(continued)

| | Units | Batch |
|---|---|---|
| **Total Solvents** | **Kg** | **210.0** |
| **Total Spray Solution Weight** | **Kg** | **260.9** |

The mixture was maintained at a temperature of 20 - 30 °C and mixed until it was substantially homogenous and all components were substantially dissolved.

A spray drier, Niro Production Minor Spray Dryer, fitted with pressure nozzles (Spray Systems Maximum Passage series SK-MFP having orifice size 72), was used under normal spray drying mode, following the dry spray process parameters recited in Table C2, below. The spray nozzle was situated approximately 5 cm from the top of the spray drying vessel.

Table C2: Dry spray process parameters used to generate Intermediate C.

| **Parameter** | **Target Value** |
|---|---|
| Feed Pressure | 30 - 100 bar |
| Feed Flow Rate | 15 - 25 Kg/hr |
| Inlet Temperature | 85 - 125 °C |
| Outlet Temperature | 45 - 75 °C |
| Vacuum Dryer Temperature | 55 °C (+/-5 °C) |
| Vacuum Drying Time | 24 hours |

A high efficiency cyclone separated the wet product from the spray gas and solvent vapors. The wet product was transferred to a tray vacuum dryer for drying to reduce residual solvents to a level of less than about 5000 ppm and to generate dry Intermediate C.

Intermediate D:

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt% hypromellose acetate succinate /0.5 wt% SLS / 80 wt% N-[2,4-Bis(1,1-dimethylethyl)-5-hydroxyphenyl]-1,4-dihydro-4-oxoquinoline-3-carboxamide. The resulting mixture contained 12.5 wt% solids. The actual amounts of ingredients and solvents used to generate this mixture are recited in Table D1, below:

Table D1: Solid Spray Dispersion Ingredients for Intermediate D.

| | Units | Batch |
|---|---|---|
| N-[2,4-Bis(1,1-dimethylethyl)-5-hydroxyphenyl]-1,4-dihydro-4-oxoquinoline-3-carboxamide | Kg | 1.60 |
| HPMCAS | Kg | 0.390 |
| SLS | Kg | 0.010 |
| **Total Solids** | **Kg** | **2.00** |
| MEK | Kg | 12.6 |
| Water | Kg | 1.40 |
| **Total Solvents** | **Kg** | **14.0** |
| **Total Spray Solution Weight** | **Kg** | **16.0** |

The mixture was maintained at a temperature of 20 - 30 °C and mixed until it was substantially homogenous and all components were substantially dissolved.

A spray drier, Niro Mobil Minor Spray Dryer fitted with a 1.0mm two fluid nozzle, was used in normal spray drying mode, following the dry spray process parameters recited in Table D2, below.

Table D2: Dry spray process parameters used to generate Intermediate D.

| **Parameter** | **Value** |
|---|---|
| Atomization Ratio | 1.5 |
| Feed Flow Rate | 4.5 - 5.0 Kg/hr |
| Outlet Temperature | 60°C |
| Vacuum Dryer Temperature | 55 °C (+/-5 °C) |
| Vacuum Drying Time | 192 hours |

A high efficiency cyclone separated the wet product from the spray gas and solvent vapors. The wet product contained 6.3% MEK and 0.7% Water and had a mean particle size of 7um and a bulk density of 0.23g/cc. The wet product was transferred to a tray vacuum dryer for drying to reduce residual solvents to a level of less than about 5000 ppm and to generate dry Intermediate D. The dry Intermediate D contained <0.5% MEK and 0.3% Water.

Intermediate E:

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt% hypromellose acetate succinate / 0.5 wt% SLS / 80 wt% N-[2,4-Bis(1,1-dimethylethyl)-5-hydroxyphenyl]-1,4-dihydro-4-oxoquinoline-3-carboxamide. The resulting mixture contained 10.5 wt% solids. The actual amounts of ingredients and solvents used to generate this mixture are recited in Table E1, below:

Table E1: Solid Spray Dispersion Ingredients for Intermediate E.

| | Units | Batch |
|---|---|---|
| N-[2,4-Bis( 1,1-dimethylethyl)-5-hydroxyphenyl]-1,4-dihdro-4-oxouinoline-3-carboxamide | Kg | 43.93 |
| HPMCAS | Kg | 10.72 |
| SLS | Kg | 0.2750 |
| **Total Solids** | **Kg** | **54.93** |
| **MEK** | Kg | 421.8 |
| Water | Kg | 46.90 |
| **Total Solvents** | **Kg** | **468.7** |
| **Total Spray Solution Weight Kg 523.6** | | |

The mixture temperature was adjusted to a range of 30 - 45 °C and mixed until it was substantially homogenous and all components were substantially dissolved.

A spray drier, Niro PSD4 Commercial Spray Dryer, fitted with pressure nozzles (Spray Systems Maximum Passage series SK-MFP having orifice/core size 54/21, 53/21 or 52/21) equipped with anti-bearding cap, was used under normal spray drying mode, following the dry spray process parameters recited in Table E2, below.

Table E2: Dry spray process parameters used to generate Intermediate E.

| **Parameter** | **Value** |
|---|---|
| Feed Pressure | 20 - 40 bar |

(continued)

| Parameter | Value |
|---|---|
| Feed Flow Rate | 90 - 160 Kg/hr |
| Inlet Temperature | 75 - 125 °C |
| Outlet Temperature | 35 - 55 °C |
| Vacuum Dryer Temperature | 80 °C (+/-5 °C) |
| Vacuum Drying Time | 156 hours |

A high efficiency cyclone separated the wet product from the spray gas and solvent vapors. The wet product contained 8.8 - 12.5%wt. MEK/Water a mean particle size of 16 - 24um and a bulk density of 0.28 - 0.36g/cc. The wet product was transferred to a 350L stainless steel double cone vacuum dryer for drying to reduce residual solvents to a level of less than about 5000 ppm and to generate dry Intermediate E. The dry Intermediate E contained <0.3% MEK and 0.8% Water.

Intermediate F:

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt% hypromellose acetate succinate / 0.5 wt% SLS / 80 wt% N-[2,4-Bis(1,1-dimethylethyl)-5-hydroxyphenyl]-1,4-dihydro-4-oxoquinoline-3-carboxamide. The resulting mixture contained 10.5 wt% solids. The actual amounts of ingredients and solvents used to generate this mixture are recited in Table F1, below:

Table F1: Solid Spray Dispersion Ingredients for Intermediate F.

| | Units | Batch |
|---|---|---|
| N-[2,4-Bis(1,1-dimethylethyl)-5-hydroxyphenyl]-1,4-dihvdro-4-oxoquinoline-3-carboxamide | Kg | 70.0 |
| HPMCAS | Kg | 17.1 |
| SLS | Kg | 0.438 |
| **Total Solids** | **Kg** | **87.5** |
| MEK | Kg | 671 |
| Water | Kg | 74.6 |
| **Total Solvents** | **Kg** | **746** |
| **Total Spray Solution Weight** | **Kg** | **833** |

The mixture temperature was adjusted to a range of 20 - 45 °C and mixed until it was substantially homogenous and all components were substantially dissolved.

A spray drier, Niro PSD4 Commercial Spray Dryer, fitted with pressure nozzle (Spray Systems Maximum Passage series SK-MFP having orifice/core size 54/21) equipped with anti-bearding cap, was used under normal spray drying mode, following the dry spray process parameters recited in Table F2, below.

Table F2: Dry spray process parameters used to generate Intermediate F.

| Parameter | Value |
|---|---|
| Feed Pressure | 20 bar |
| Feed Flow Rate | 92 - 100 Kg/hr |
| Inlet Temperature | 93 - 99 °C |

(continued)

| Parameter | Value |
|---|---|
| Outlet Temperature | 53 - 57 °C |
| Vacuum Dryer Temperature | 80 °C for 2 hours then 110 °C (+/-5 °C) |
| Vacuum Drying Time | 20 - 24 hours |

A high efficiency cyclone separated the wet product from the spray gas and solvent vapors. The wet product contained 8.5 - 9.7% MEK and 0.56 - 0.83% Water and had a mean particle size of 17 - 19um and a bulk density of 0.27 - 0.33g/cc. The wet product was transferred to a 4000L stainless steel double cone vacuum dryer for drying to reduce residual solvents to a level of less than about 5000 ppm and to generate dry Intermediate F. The dry Intermediate F contained <0.03% MEK and 0.3% Water.

Intermediate G:

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt % hypromellose acetate succinate / 0.5 wt % SLS / 80 wt% N-[2,4-Bis(1,1-dimethylethyl)-5-hydroxyphenyl]-1,4-dihydro-4-oxoquinoline-3-carboxamide. The resulting mixture contained 10.5 wt% solids. The actual amounts of ingredients and solvents used to generate this mixture are recited in Table G1, below:

Table G1: Solid Spray Dispersion Ingredients for Intermediate G.

| | Units | Batch |
|---|---|---|
| N-[2,4-Bis(1,1-dimethylethyl)-5-hydroxyphenyl]-1,4-dihydro-4-oxoquinoline-3-carboxamide | Kg | 24.0 |
| HPMCAS | Kg | 5.85 |
| SLS | Kg | 0.15 |
| **Total Solids** | **Kg** | **30.0** |
| **MEK** | Kg | 230.1 |
| Water | Kg | 25.6 |
| **Total Solvents** | **Kg** | **255.7** |
| **Total Spray Solution Weight** | **Kg** | **285.7** |

The mixture temperature was adjusted to a range of 20 - 45 °C and mixed until it was substantially homogenous and all components were substantially dissolved.

A spray drier, Niro Production Minor Spray Dryer, fitted with pressure nozzle (Spray Systems Maximum Passage series SK-MFP having orifice size 72) was used under normal spray drying mode, following the dry spray process parameters recited in Table G2, below.

Table G2: Dry spray process parameters used to generate Intermediate G.

| Parameter | Value |
|---|---|
| Feed Pressure | 33 bar |
| Feed Flow Rate | 18 - 24 Kg/hr |
| Inlet Temperature | 82 - 84 °C |
| Outlet Temperature | 44 - 46 °C |
| Vacuum Dryer Temperature | 80 °C for 2 hours then 110 °C (+/-5 °C) |

(continued)

| Parameter | Value |
|---|---|
| Vacuum Drying Time | 48 hours |

A high efficiency cyclone separated the wet product from the spray gas and solvent vapors. The wet product contained 10.8% MEK and 0.7% Water and had a mean particle size of 19um and a bulk density of 0.32g/cc. The wet product was transferred to a 4000L stainless steel double cone vacuum dryer for drying to reduce residual solvents to a level of less than about 5000 ppm and to generate dry Intermediate. The dry Intermediate G contained <0.05% MEK and 0.7% Water.

Example 1: Exemplary Tablet 1 (Formulated to have 25 mg of Compound 1)

A batch of round core 3/8" tablets was formulated to have approximately 25 mg of Compound 1 per tablet using the amounts of ingredients recited in Table 1, below.

Table 1: Ingredients for Exemplary Tablet 1.

| Tablet Formulation | Percent Dose %Wt./Wt. | Dose (mg) | Batch (g) |
|---|---|---|---|
| Intermediate A | 15.29% | 51.23 | 512.5 |
| Microcrystalline cellulose | 35.00% | 117.25 | 1172 |
| Lactose | 43.85% | 146.00 | 1460 |
| Sodium croscarmellose | 5.000% | 16.75 | 167.5 |
| SLS | 0.500% | 1.675 | 16.75 |
| Colloidal silicon dioxide | 0.125% | 0.4188 | 4.188 |
| Magnesium stearate | 0.50% | 1.675 | 16.75 |
| **Total** | **100%** | **335** | **3350** |

Intermediate A, microcrystalline cellulose (FMC MCC Avicel® PH102, commercially available from FMC BioPolymer Corporation of Philadelphia, PA), lactose (Foremost FastFlo® Lactose #316 commercially available from Foremost Farms USA of Baraboo, WI), sodium croscarmellose (FMC Ac-Di-Sol®, commercially available from FMC BioPolymer Corporation of Philadelphia, PA), SLS, and colloidal silicon dioxide (Cabot Cab-O-Sil® M-5P Fumed Silicon Dioxide, commercially available from Cabot Corporation of Alpharetta, GA) were sieved through a 20 mesh screen to remove lumps.

Each of the sieved ingredients was added to a 16 quart V-blender in the following order:

1) lactose;
2) SLS;
3) sodium croscarmellose;
4) colloidal silicon dioxide;
5) Intermediate A; and
6) microcrystalline cellulose PH101

The mixture was blended for 25 minutes in a V-blender at 20-24 rpm. Magnesium stearate was sieved through a 30 mesh screen to remove lumps, and added to the mixture, which was blended for another 3 minutes.

Once the final blend has been completed, the mixture was transferred to a Piccola B-Tooling, 10 Station rotary tablet press (half tooled) for compression. Pressing the mixture into tablets generated 3/8" round tablets having approximately 25 mg of N-[2,4-Bis(1,1-dimethylethyl)-5-hydroxyphenyl]-1,4-dihydro-4-oxoquinoline-3-carboxamide.

Example 2: Exemplary Tablet 2 (Formulated to have 50 mg of Compound 1)

A batch of round core 3/8" tablets was formulated to have about 50 mg of Compound 1 per tablet using the amounts of ingredients recited in Table 2, below.

Table 2: Ingredients for Exemplary Tablet 2.

| Tablet Formulation | Percent Dose % Wt./Wt. | Dose (mg) | Batch (g) |
|---|---|---|---|
| Intermediate A | 30.60% | 102.50 | 1025.0 |
| Microcrystalline cellulose | 25.00% | 83.75 | 837.5 |
| Lactose | 38.28% | 128.23 | 1282.3 |
| Sodium croscarmellose | 5.000% | 16.75 | 167.5 |
| SLS | 0.500% | 1.675 | 16.75 |
| Colloidal silicon dioxide | 0.125% | 0.4188 | 4.188 |
| Magnesium stearate | 0.50% | 1.675 | 16.75 |
| **Total** | **100%** | **335** | **3350** |

Intermediate A, microcrystalline cellulose, lactose, sodium croscarmellose, SLS, and colloidal silicon dioxide were sieved through a 20 mesh screen to remove lumps, and each of the sieved ingredients was added to a 16 quart V-blender in the following order:

1) lactose;
2) SLS;
3) sodium croscarmellose;
4) colloidal silicon dioxide;
5) Intermediate A; and
6) microcrystalline cellulose PH101

The mixture was blended for 25 minutes in a V-blender at 20-24 rpm. Magnesium stearate was sieved through a 30 mesh screen to remove lumps, and added to the mixture, which was blended for another 3 minutes.

Once the final blend has been completed, the mixture was transferred to a Piccola B-Tooling, 10 Station rotary tablet press (half tooled) for compression. Pressing the mixture into tablets generated 3/8" round tablets having approximately 50 mg of N-[2,4-Bis(1,1-dimethylethyl)-5-hydroxyphenyl]-1,4-dihydro-4-oxoquinoline-3-carboxamide.

Example 3: Exemplary Tablet 3 (Formulated with PVP/VA Polymer to have 150 mg of Compound 1)

A batch of caplet-shaped tablets was formulated to have about 150 mg of Compound 1 per tablet using the amounts of ingredients recited in Table 3, below.

Table 3: Ingredients for Exemplary Tablet 3.

| Tablet Formulation | Percent Dose %Wt./Wt. | Dose (mg) | Batch (g) |
|---|---|---|---|
| Intermediate B | 40.000% | 187.50 | 240.00 |
| Microcrystalline cellulose | 27.063% | 126.86 | 162.38 |
| Lactose | 27.063% | 126.86 | 162.38 |
| Sodium croscarmellose | 3.000% | 14.06 | 18.00 |
| SLS | 0.500% | 2.34 | 3.00 |
| Colloidal silicon dioxide | 1.000% | 4.69 | 6.00 |
| Coloring | 0.375% | 1.76 | 2.25 |
| Magnesium stearate | 1.000% | 4.69 | 6.00 |
| **Total** | **100%** | **469** | **600** |

A glidant blend of colloidal silicon dioxide (Cabot Cab-O-Sil® M-5P Fumed Silicon Dioxide) and SLS was produced by hand mixing these two ingredients, in the amounts given in Table 3, and filtering the resulting mix through

a 70 mesh screen sieve. A color blend of coloring (Colorcon Blue #1 Aluminum Lake #5516) and sodium croscarmellose (FMC Ac-Di-Sol®) was produced by hand mixing these two ingredients, in the amounts given in Table 3, and filtering the resulting mix through a 70 mesh screen sieve. The glidant blend and the color blend were hand mixed and added to a 2 L blending container. Intermediate B was added to this mixture in the 2 L blending container, and the contents 2 L blending container were hand mixed and filtered through a 30 mesh screen sieve. The resulting mixture was mixed on a Turbula mixer for 20 minutes at a rate of 22 rpm.

The microcrystalline cellulose (FMC MCC Avicel® PH102) and lactose (Foremost FastFlo® Lactose #316) were each filtered through a 30 mesh screen sieve and added to the blending container. The resulting mixture was mixed on a Turbula mixer for 20 minutes at a rate of 22 rpm.

Magnesium Stearate was filtered through a 70 mesh screen sieve and added to the mixture in the blending container, and the resulting mixture was mixed for 5 minutes at a rate of 22 rpm.

The resulting mixture was compressed into tablets using a gravity fed boot tooled with 0.64" × 0.32" caplet type B tooling set to produce a tablet having an initial hardness of about 8 Kp ± 15%.

Example 4: Exemplary Tablet 4 (Formulated with HPMCAS Polymer to have 150 mg of Compound 1)

A batch of caplet-shaped tablets was formulated to have about 150 mg of Compound 1 per tablet using the amounts of ingredients recited in Table 4, below.

Table 4: Ingredients for Exemplary Tablet 4.

| Tablet Formulation | Percent Dose %Wt./Wt. | Dose (mg) | Batch (g) |
|---|---|---|---|
| Intermediate C | 34.091 % | 187.50 | 204.55 |
| Microcrystalline cellulose | 30.017% | 165.09 | 180.10 |
| Lactose | 30.017% | 165.09 | 180.10 |
| Sodium croscarmellose | 3.000% | 16.50 | 18.00 |
| SLS | 0.500% | 2.75 | 3.00 |
| Colloidal silicon dioxide | 1.000% | 5.50 | 6.00 |
| Coloring | 0.375% | 2.06 | 2.25 |
| Magnesium stearate | 1.000% | 5.50 | 6.00 |
| | | | |
| Total | 100% | 550 | 600 |

A glidant blend of colloidal silicon dioxide (Cabot Cab-O-Sil® M-5P Fumed Silicon Dioxide) and SLS was produced by hand mixing these two ingredients, in the amounts given in Table 4, and filtering the resulting mix through a 70 mesh screen sieve. A color blend including coloring (Colorcon Blue #1 Aluminum Lake #5516) and sodium croscarmellose (FMC Ac-Di-Sol®) was produced by hand mixing these two ingredients, in the amounts given in Table 4, and filtering the resulting mix through a 70 mesh screen sieve. The glidant blend and the color blend were hand mixed and added to a 2 L blending container. Intermediate C was added to this mixture in the 2 L blending container, and the contents 2 L blending container were hand mixed and filtered through a 30 mesh screen sieve. The resulting mixture was mixed on a Turbula mixer for 20 minutes at a rate of 22 rpm.

The microcrystalline cellulose (FMC MCC Avicel® PH102) and lactose (Foremost FastFlo® Lactose #316) were each filtered through a 30 mesh screen sieve and added to the blending container. The resulting mixture was mixed on a Turbula mixer for 20 minutes at a rate of 22 rpm.

Magnesium stearate was filtered through a 70 mesh screen sieve and added to the mixture in the blending container, and the resulting mixture was mixed for 5 minutes at a rate of 22 rpm.

The resulting mixture was compressed into tablets using a tablet press tooled with 0.64" × 0.32" caplet type B tooling set to produce a tablet having an initial hardness of about 9.5 Kp ± 15%.

Example 5: Exemplary Tablet 5 (Formulated with HPMCAS Polymer to have 150 mg of Compound 1)

A batch of caplet-shaped tablets was formulated to have about 150 mg of Compound 1 per tablet using the amounts of ingredients recited in Table 5, below.

Table 5: Ingredients for Exemplary Tablet 5.

| Tablet Formulation | Percent Dose %Wt./Wt. | Dose (mg) | Batch (g) |
|---|---|---|---|
| Intermediate G | 34.564% | 190.10 | 21000.00 |
| Microcrystalline cellulose | 29.968% | 164.82 | 18207.62 |
| Lactose | 29.968% | 164.82 | 18207.62 |
| Sodium croscarmellose | 3.000% | 16.50 | 1822.71 |
| SLS | 0.500% | 2.75 | 303.78 |
| Colloidal silicon dioxide | 1.000% | 5.50 | 607.57 |
| Magnesium stearate | 1.000% | 5.50 | 607.57 |
| **Total** | **100%** | **550** | **607560** |

A blend of colloidal silicon dioxide (Cabot Cab-O-Sil® M-5P Fumed Silicon Dioxide), SLS, sodium croscarmellose (FMC Ac-Di-Sol®), and approximately 10% of the lactose (Foremost FastFlo® Lactose #316) given in Table 5 was produced by mixing these ingredients in a V-blender to provide about 125 inversions. This mixture, Preblend 1, was cone-milled through a 40 mesh screen sieve, collected and stored for subsequent use.

Approximately 20% of the lactose (Foremost FastFlo® Lactose #316) given in Table 5 was cone-milled through a 30 mesh screen sieve, collected and stored for subsequent use as Preblend 2. Intermediate G was filtered through a 30 mesh screen, collected and stored for subsequent use as Preblend 3. The microcrystalline cellulose (FMC MCC Avicel® PH102) was filtered through a 30 mesh screen, collected and stored for subsequent use as Preblend 4.

A V-blender was charged with Preblend 2, the remaining 70% of the lactose (Foremost FastFlo® Lactose #316) given in Table 3, Preblend 3, Preblend 1, and Preblend 4, in that order, and blended for about 500 inversions. The blended mixture was tested for uniformity.

Magnesium Stearate was filtered through a 70 mesh screen sieve and added to the mixture in the blending container, and the resulting mixture was mixed to provide about 125 inversions.

The resulting mixture was compressed into tablets using a Killian T100 press tooled with 0.64" $\times$ 0.32" caplet type B tooling set to produce a tablet having an initial hardness of about 11 Kp $\pm$ 20%.

Example 6: Exemplary Tablet 6 (Formulated with HPMCAS Polymer to have 100 mg of Compound 1)

A batch of caplet-shaped tablets was formulated to have about 150 mg of Compound 1 per tablet using the amounts of ingredients recited in Table 6, below.

Table 6: Ingredients for Exemplary Tablet 6.

| Tablet Formulation | Percent Dose %Wt./Wt. | Dose (mg) | Batch (g) |
|---|---|---|---|
| Intermediate G | 34.564% | 126.73 | 9000.06 |
| Microcrystalline cellulose | 29.968% | 109.88 | 7803.32 |
| Lactose | 29.968% | 109.88 | 7803.32 |
| Sodium croscarmellose | 3.000% | 11.00 | 781.17 |
| SLS | 0.500% | 1.83 | 130.19 |
| Colloidal silicon dioxide | 1.000% | 3.67 | 260.39 |
| Magnesium stearate | 1.000% | 3.67 | 260.39 |
| **Total** | **100%** | **367** | **26040** |

A blend of colloidal silicon dioxide (Cabot Cab-O-Sil® M-5P Fumed Silicon Dioxide), SLS, sodium croscarmellose (FMC Ac-Di-Sol®), and approximately 10% of the lactose (Foremost FastFlo® Lactose #316) given in Table 6 was produced by mixing these ingredients in a V-blender to provide about 125 inversions. This mixture, Preblend 1, was cone-milled through a 40 mesh screen sieve, collected and stored for subsequent use.

Approximately 20% of the lactose (Foremost FastFlo® Lactose #316) given in Table 6 was cone-milled through

a 30 mesh screen sieve, collected and stored for subsequent use as Preblend 2. Intermediate G was filtered through a 30 mesh screen, collected and stored for subsequent use as Preblend 3. The microcrystalline cellulose (FMC MCC Avicel® PH102) was filtered through a 30 mesh screen, collected and stored for subsequent use as Preblend 4.

A V-blender was charged with Preblend 2, the remaining 70% of the lactose (Foremost FastFlo® Lactose #316) given in Table 3, Preblend 3, Preblend 1, and Preblend 4, in that order, and blended for about 500 inversions. The blended mixture was tested for uniformity.

Magnesium Stearate was filtered through a 70 mesh screen sieve and added to the mixture in the blending container, and the resulting mixture was mixed to provide about 125 inversions.

The resulting mixture was compressed into tablets using a Killian T100 press tooled with 0.64" × 0.32" caplet type B tooling set to produce a tablet having an initial hardness of about 11 Kp ± 20%.

Example 7: Exemplary Tablets 7 and 8 (Tablet 5 and 6 with Spray-Coating

A batch of caplet-shaped tablets from Example 5 and 6 was spray-coated with OPADRY® II (Blue, Colorcon) to a weight gain of about 3.0% using a 24" coating pan configured with the parameters in Table 7 followed by logo printing using Opacode® WB (Black, Colorcon).

Table 7: Spray-Coating Process Parameters

| Coating Parameters 24" Pan | Target |
|---|---|
| Pan Load (kg) | 15 |
| Inlet Temperature (°C)* | * |
| Pan Speed (rpm) | 14 |
| Jog Time | TBD |
| # of Spray Guns | 2 |
| Solids Content (%w/w) | 20 |
| Gun to Bed Distance (inches) | 6 |
| Inlet Air Flow (cfm) | 250, 300** |
| Spray Rate (g/min) | 70 |
| Exhaust Temperature (°C) | 50 |
| Atomization Pressure (psi) | 25 |
| Pattern Pressure (psi) | 25 |
| * Inlet temperature is monitored to achieve target exhaust temperature. Initial inlet temperature should be set at about 75°C to achieve target exhaust temp.<br>** The target Inlet Air Flow was 250, 300 for Tablet 7 and Tablet 8, respectively. | |

The OPADRY® II suspension was prepared by measuring an amount of de-ionized water which when combined with OPADRY® II would produce a total solids content of 20 %w/w. The water is mixed to a vortex followed by addition of OPADRY® II over a period of approximately 5 minutes. Once the OPADRY® II powder was wetted, mixing was continued to ensure that all solid material is well-dispersed. The suspension is then charged into a Thomas 24" pan coating instrument using coating conditions outlined in Table 7.

Core tablets are placed into the coating pan and pre-warmed. The inlet temperature was increased from room temperature to about 55°C and then increased as necessary to provide the exhaust temperature in Table 7. The coating process was performed with 20% w/w OPADRY® II (85 Series Blue) coating dispersion to obtain a target weight gain of about 3%. The coated tablets were then allowed to tumble for about 2 minutes without spraying. The bed temperature was then allowed to cool to about 35°C.

Once coated with OPADRY® II, the tablets are then labeled using a Hartnett Delta tablet printer charged with Opacode® WB.

Example 8: Exemplary Tablet 9 (Formulated with HPMCAS Polymer to have 100 mg of Compound 1)

A batch of caplet-shaped tablets was formulated to have about 100 mg of Compound 1 per tablet using the

amounts of ingredients recited in Table 8, below.

Table 8: Ingredients for Exemplary Tablet 9.

| Tablet Formulation | Percent Dose %Wt./Wt. | Dose (mg) | Batch (g) |
|---|---|---|---|
| Intermediate F | 34.09% | 125.1 | 23.86 |
| Microcrystalline cellulose | 30.51% | 112.0 | 21.36 |
| Lactose | 30.40% | 111.6 | 21.28 |
| Sodium croscarmellose | 3.000% | 11.01 | 2.100 |
| SLS | 0.500% | 1.835 | 0.3500 |
| Colloidal silicon dioxide | 0.500% | 1.835 | 0.3500 |
| Magnesium stearate | 1.000% | 3.670 | 0.7000 |
| **Total** | **100%** | **367** | **70** |

The colloidal silicon dioxide (Cabot Cab-O-Sil® M-5P Fumed Silicon Dioxide) and the microcrystalline cellulose (FMC MCC Avicel® PH102) were passed through a 30 mesh screen.

The sodium croscarmellose (FMC Ac-Di-Sol®), SLS, Intermediate F, and lactose (Foremost FastFlo® Lactose #316) were also passed, individually in the preceding order, through the same 30 mesh screen. A nitrogen purge was used when screening Intermediate F. The screened components were loaded into a 10 cubic feet V-blender, which was purged with nitrogen, and blended for about 180 (+/- 10) inversions.

The Magnesium Stearate was filtered through a 40 mesh screen sieve into the blending container and mixed to provide about 54 inversions.

The resulting mixture was compressed into tablets using a fully tooled 36 Fette 2090 press with 0.568" x 0.2885" caplet type B tooling set to produce a tablet having an initial target hardness of about 10 Kp ± 20%.

Example 9: Exemplary Tablet 10 (Tablet 9 with Spray-Coating)

A batch of caplet-shaped tablets from Example 8 was spray-coated with OPADRY® II (Blue, Colorcon) to a weight gain of about 3.0% using a 24" coating pan configured with the parameters in Table 9 followed by wax coating and then printing using Opacode® S-1-17823 (Solvent based Black, Colorcon).

Table 9: Spray-Coating Process Parameters

| **Coating Parameters 24" Pan** | **Target** |
|---|---|
| Pan Load (kg) | 14 |
| Inlet Temperature (°C)* | * |
| Pan Speed (rpm) | 10 |
| Jog Time (sec) | |
| # of Spray Guns | 2 |
| Solids Content (%w/w) | 20 |
| Gun to Bed Distance (inches) | 6 |
| Inlet Air Flow (cfm) | 300 |
| Spray Rate (g/min) | 35 |
| Exhaust Temperature (°C) | 50 |
| Atomization Pressure (psi) | 42 |
| * Inlet temperature is monitored to achieve target exhaust temperature. Initial inlet temperature should be set at about 75°C to achieve target exhaust temp. | |

The OPADRY® II suspension was prepared by measuring an amount of de-ionized water which when combined

with OPADRY® II would produce a total solids content of 20 %w/w. The water is mixed to a vortex followed by addition of OPADRY® II over a period of approximately 5 minutes. Once the OPADRY® II powder was wetted, mixing was continued to ensure that all solid material is well-dispersed. The suspension is then charged into a Thomas 24" pan coating instrument using coating conditions outlined in Table 9.

Uncoated tablets are placed into the coating pan and pre-warmed. The inlet was increased from room temperature to about 55°C and then increased as necessary to provide the exhaust temperature in Table 9. The coating process was performed with 20% w/w OPADRY® II (85 Series Blue) coating dispersion to obtain a target weight gain of about 3%. The coated tablets were then allowed to tumble for about 2 minutes without spraying. The bed temperature was then allowed to cool to about 35°C.

Upon cooling, the Carnauba wax powder was weighed out in the amount of about 0.01% w/w of the starting tablet core weight. With the air flow off, the carnauba wax powder was sprinkled evenly on the tablet bed. The pan bed was turned on to the speed indicated in Table 9. After 5 minutes, the air flow was turned on (without heating) to the setting indicated in Table 9. After about one minute the air flow and pan were turned off.

Once coated with OPADRY® II, the tablets are then labeled using a Hartnett Delta tablet printer charged with Opacode® S-1-17823.

Example 10: Exemplary Tablet 11 (Formulated with HPMCAS Polymer to have 150 mg of Compound 1)

A batch of caplet-shaped tablets was formulated to have about 100 mg of Compound 1 per tablet using the amounts of ingredients recited in Table 11, below.

Table 10: Ingredients for Exemplary Tablet 11.

| Tablet Formulation | Percent Dose %Wt./Wt. | Dose (mg) | Batch (g) |
|---|---|---|---|
| Intermediate F | 34.09% | 187.5 | 23.86 |
| Microcrystalline cellulose | 30.51% | 167.8 | 21.36 |
| Lactose | 30.40% | 167.2 | 21.28 |
| Sodium croscarmellose | 3.000% | 16.50 | 2.100 |
| SLS | 0.500% | 2.750 | 0.3500 |
| Colloidal silicon dioxide | 0.500% | 2.750 | 0.3500 |
| Magnesium stearate | 1.000% | 5.500 | 0.7000 |
| **Total** | **100%** | **550** | **70** |

The colloidal silicon dioxide (Cabot Cab-O-Sil® M-5P Fumed Silicon Dioxide) and the microcrystalline cellulose (FMC MCC Avicel® PH102) were passed through a 30 mesh screen.

The sodium croscarmellose (FMC Ac-Di-Sol®), SLS, Intermediate F, and lactose (Foremost FastFlo® Lactose #316) were also passed, individually in the preceding order, through the same 30 mesh screen. A nitrogen purge was used when screening Intermediate F. The screened components were loaded into a 10 cubic feet V-blender, which was purged with nitrogen, and blended for about 180 (+/- 10) inversions.

The Magnesium Stearate was filtered through a 40 mesh screen sieve into the blending container and mixed to provide about 54 inversions.

The resulting mixture was compressed into tablets using a fully tooled 36 Fette 2090 press with 0.568" x 0.2885" caplet type B tooling set to produce a tablet having an initial target hardness of about 10 Kp ± 20%.

Example 11: Exemplary Tablet 12 (Tablet 11 with Spray-Coating)

A batch of caplet-shaped tablets from Example 10 was spray-coated with OPADRY® II (Blue, Colorcon) to a weight gain of about 3.0% using a 24" coating pan configured with the parameters in Table 11 followed by wax coating and then printing using Opacode® S-1-17823 (Solvent based Black, Colorcon).

Table 11: Spray-Coating Process Parameters

| **Coating Parameters 24" Pan** | **Target** |
|---|---|
| Pan Load (kg) | 14 |

(continued)

| Coating Parameters 24" Pan | Target |
|---|---|
| Inlet Temperature (°C)* | * |
| Pan Speed (rpm) | 10 |
| Jog Time (sec) | 2-5 sec every 60 sec |
| # of Spray Guns | 2 |
| Solids Content (%w/w) | 20 |
| Gun to Bed Distance (inches) | 6 |
| Inlet Air Flow (cfm) | 300 |
| Spray Rate (g/min) | 35 |
| Exhaust Temperature (°C) | 50 |
| Atomization Pressure (psi) | 42 |
| * Inlet temperature is monitored to achieve target exhaust temperature. Initial inlet temperature should be set at about 75°C to achieve target exhaust temp. | |

The OPADRY® II suspension was prepared by measuring an amount of de-ionized water which when combined with OPADRY® II would produce a total solids content of 20 %w/w. The water is mixed to a vortex followed by addition of OPADRY® II over a period of approximately 5 minutes. Once the OPADRY@ II powder was wetted, mixing was continued to ensure that all solid material is well-dispersed. The suspension is then charged into a Thomas 24" pan coating instrument using coating conditions outlined in Table 11.

Uncoated tablets are placed into the coating pan and pre-warmed. The inlet was increased from room temperature to about 55°C and then increased as necessary to provide the exhaust temperature in Table 9. The coating process was performed with 20% w/w OPADRY® II (85 Series Blue) coating dispersion to obtain a target weight gain of about 3%. The coated tablets were then allowed to tumble for about 2 minutes without spraying. The bed temperature was then allowed to cool to about 35°C.

Upon cooling, the Carnauba wax powder was weighed out in the amount of about 0.01% w/w of the starting tablet core weight. With the air flow off, the carnauba wax powder was sprinkled evenly on the tablet bed. The pan bed was turned on to the speed indicated in Table 11. After 5 minutes, the air flow was turned on (without heating) to the setting indicated in Table 11. After about one minute the air flow and pan were turned off.

Once coated with OPADRY® II, the tablets are then labeled using a Hartnett Delta tablet printer charged with Opacode® S-1-17823.

### B. Administration of Pharmaceutical Formulations

Example 12: Exemplary Administration A

Human patients are orally administered a pharmaceutical formulation according to Table 12:

Table 12: Exemplary administration A of pharmaceutical formulations of the present invention.

| Frequency of dosing (per day) | Tablet Description | Conditions |
|---|---|---|
| One administration | 3x50 mg Tablets of Example 2 | Administered with 240 mL of water under fasting conditions |
| One administration | 150 mg Tablet of Example 3 | Administered with 240 mL of water under fasting conditions |
| One administration | 150 mg Tablet of Example 3 | Administered with 240 mL of water, 30 minutes after start of a high fat breakfast |
| One administration | 150 mg Tablet of Example 4 | Administered with 240 mL of water under fasting conditions |
| One administration | 150 mg Tablet of Example 4 | Administered with 240 mL of water, 30 minutes after start of a high fat breakfast |

The pharmaceutical formulations are administered to subjects between 7:00 AM and 9:00 AM, and the pharmaceutical formulation is given at approximately the same time (within a 1-hour window) on each dosing occasion. For administrations that occur under patient fasting, food is allowed 4 hours after the pharmaceutical formulation is administered. For administrations that permit feeding, breakfast is given about 30 minutes prior to the dosing time and is consumed in about 25 minutes. In each of these administrations, the patient is instructed not to lie down for 4 hours after taking the study drug.

Example 13: Exemplary Administration **B**

Human patients are orally administered a pharmaceutical formulation according to Table 13:

Table 13: Exemplary administration B of pharmaceutical formulations of the present invention.

| Frequency of dosing | Dosage |
|---|---|
| 12 hr. intervals | 25 mg Tablet of Example 1 |
| 12 hr. intervals | 1×25 mg Tablet of Example 1, and 1×50 mg Tablet of Example 2 |
| 12 hr. intervals | 3×50 mg Tablet of |
| | Example 2 |
| 12 hr. intervals | 5×50 mg Tablet of Example 2 |
| 12 hr. intervals | 150 mg Tablet of Example 5 |
| 12 hr. intervals | 100 mg Tablet of Example 6 |

The pharmaceutical formulations are administered to patients approximately every 12 hours.

Example 14: Dissolution Profile of Several Exemplary Tablets

Referring to Figure 1, the dissolution profiles of several exemplary tablets are graphically illustrated. It is noted that each of the exemplary tablets illustrated in Figure 1 are at least 50% dissolved at about 30 minutes.

**OTHER EMBODIMENTS**

All publications and patents referred to in this disclosure are incorporated herein by reference to the same extent as if each individual publication or patent application were specifically and individually indicated to be incorporated by reference. Should the meaning of the terms in any of the patents or publications incorporated by reference conflict with the meaning of the terms used in this disclosure, the meaning of the terms in this disclosure are intended to be controlling. Furthermore, the foregoing discussion discloses and describes merely exemplary embodiments of the present invention. One skilled in the art will readily recognize from such discussion and from the accompanying drawings and claims, that various changes, modifications and variations can be made therein without departing from the spirit and scope of the invention as defined in the following claims.

The invention is further defined with reference to the following clauses:

1. A pharmaceutical composition comprising:

   a. a solid dispersion comprising substantially amorphous Compound 1 and a polymer;
   b. a filler;
   c. a disintegrant;
   d. a surfactant;
   e. a binder;
   f. a glidant; and
   g. a lubricant.

2. The pharmaceutical composition of clause 1, wherein the solid dispersion comprises substantially amorphous Compound 1 and a polymer, and the polymer comprises HPMC, HPMCAS, PVP/VA, PVP, methacrylic acid/methacrylate copolymer, HPC, or any combination thereof.

3. The pharmaceutical composition of clause 2, wherein the solid dispersion has a mean particle diameter of greater than about 5 μm.

4. The pharmaceutical composition of clause 2, wherein the solid dispersion has a bulk density of about 0.10 g/cc or greater.

5. The pharmaceutical composition of clause 2, wherein the solid dispersion comprises substantially amorphous Compound 1, and Compound 1 is present in a concentration of at least 20 wt% by weight of the solid dispersion.

6. The pharmaceutical composition of clause 5, wherein the solid dispersion comprises 80 wt% or less of HPMCAS or PVP/VA.

7. The pharmaceutical composition of clause 5, wherein the solid dispersion comprises a surfactant.

8. The pharmaceutical composition of clause 7, wherein the solid dispersion comprises less than 10 wt% of surfactant by weight of solid dispersion.

9. The pharmaceutical composition of clause 8, wherein the solid dispersion comprises a surfactant, and the surfactant is SLS.

10. The pharmaceutical composition of clause 9, wherein the solid dispersion comprises amorphous Compound 1.

11. The pharmaceutical composition of clause 1, wherein the solid dispersion comprises from about 45 wt% to about 85 wt% of substantially amorphous Compound 1, from about 0.45 wt% to about 0.55 wt% of SLS, and from about 14.45 wt% to about 55.55 wt% of HPMCAS or PVP/VA by weight of the solid dispersion.

12. The pharmaceutical composition of clause 1, wherein the solid dispersion comprises from about 40 wt% to about 60 wt% of substantially amorphous Compound 1 by weight of the solid dispersion and from about 60 wt% to about 40 wt% of polymer by weight of the solid dispersion.

13. The pharmaceutical composition of clause 1, wherein the solid dispersion comprises from about 65 wt% to about 95 wt% of substantially amorphous Compound 1 by weight of the solid dispersion and from about 45 wt% to about 5 wt% of polymer by weight of the solid dispersion.

14. The pharmaceutical composition of any of clauses 1-13, wherein the filler is lactose, sorbitol, cellulose, calcium phosphate, starch, sugar, or any combination thereof.

15. The pharmaceutical composition of any of clauses 1-14, wherein the filler is lactose and has a concentration of at least about 10 wt% by weight of the composition.

16. The pharmaceutical composition of any of clauses 1-15, wherein the disintegrant is sodium croscarmellose, sodium starch glycolate, or a combination thereof.

17. The pharmaceutical composition of any of clauses 1-16, wherein the disintegrant is sodium croscarmellose and has a concentration of about 10 wt% or less by weight of the composition.

18. The pharmaceutical composition of any of clauses 1-17, wherein the surfactant is sodium lauryl sulfate, sodium stearyl fumarate, polyoxyethylene 20 sorbitan mono-oleate, or any combination thereof.

19. The pharmaceutical composition of any of clauses 1-18, wherein the surfactant is sodium lauryl sulfate and has a concentration of about 10 wt% or less by weight of the composition.

20. The pharmaceutical composition of any of clauses 1-19, wherein the binder is microcrystalline cellulose, dibasic calcium phosphate, sucrose, corn starch, modified cellulose, or any combination thereof.

21. The pharmaceutical composition of any of clauses 1-20, wherein the binder is microcrystalline cellulose and has a concentration of at least about 1 wt% by weight of the composition.

22. The pharmaceutical composition of any of clauses 1-21, wherein the glidant is colloidal silicon dioxide, talc, or a combination thereof.

23. The pharmaceutical composition of any of clauses 1-22, wherein the glidant is colloidal silicon dioxide and has a concentration of 2 wt% or less by weight of the composition.

24. The pharmaceutical composition of any of clauses 1-23, wherein the lubricant is magnesium stearate, stearic acid, hydrogenated oil, sodium stearyl fumarate, or any combination thereof.

25. The pharmaceutical composition of any of clauses 1-24, wherein the lubricant is magnesium stearate and has a concentration of about 2 wt% by weight of the composition.

26. The pharmaceutical composition of any of clauses 1-25, further comprising a colorant.

27. The pharmaceutical composition of any of clauses 1-26, wherein the colorant is a blue pigment having a concentration of less than 1 wt% by weight of the composition.

28. A pharmaceutical composition comprising
from about 5 wt% to about 50 wt% of a solid dispersion, by weight of the composition, comprising from about 40 wt% to about 60 wt% of substantially amorphous Compound 1, by weight of the dispersion, and from about 60 wt% to about 40 wt% of a polymer, by weight of the dispersion;

b. from about 25 wt% to about 50 wt% of a filler;
c. from about 1 wt% to about 10 wt% of a disintegrant;
d. from about 2 wt% to about 0.3 wt% of a surfactant;
e. from about 5 wt% to about 50 wt% of a binder;
f. from about 2 wt% to about 0.05 wt% of a glidant; and
h. from about 2 wt% to about 0.1 wt% of a lubricant.

29. A pharmaceutical composition comprising
from about 5 wt% to about 50 wt% of a solid dispersion, by weight of the composition, comprising from about 70 wt% to about 90 wt% of substantially amorphous Compound 1, by weight of the dispersion, and from about 30 wt% to about 10 wt% of a polymer, by weight of the dispersion;

b. from about 25 wt% to about 50 wt% of a filler;
c. from about 1 wt% to about 10 wt% of a disintegrant;
d. from about 2 wt% to about 0.3 wt% of a surfactant;
e. from about 5 wt% to about 50 wt% of a binder;
f. from about 2 wt% to about 0.05 wt% of a glidant; and
h. from about 2 wt% to about 0.1 wt% of a lubricant.

30. The pharmaceutical composition of any of clauses 1-29, further comprising a tablet, a capsule, or a suspension.

31. The pharmaceutical composition of clause 30, further comprising a tablet, and the tablet has a hardness of at least 5 Kp.

32. A pharmaceutical composition consisting of a tablet that comprises a solid dispersion, a filler, a disintegrant, a surfactant, a binder, a glidant, and a lubricant, wherein the tablet has a dissolution of at least about 50% in about 30 minutes, and the solid dispersion comprises substantially amorphous Compound 1.

33. The pharmaceutical composition of clause 32, wherein the solid dispersion comprises substantially amorphous Compound 1 and HPMCAS or PVP/VA.

34. The pharmaceutical composition of clause 33, wherein the solid dispersion comprises at least about 25 mg of substantially amorphous Compound 1, and PVP/VA and SLS.

35. The pharmaceutical composition of clause 33, wherein the solid dispersion comprises at least about 25 mg of substantially amorphous Compound 1, and HPMCAS and SLS.

36. A pharmaceutical composition consisting of a tablet that comprises

a. a solid dispersion comprising substantially amorphous Compound 1, and HPMCAS or PVP/VA;
b. a filler;
c. a disintegrant;
d. a surfactant;
e. a binder;
f. a glidant; and
g. a lubricant.

37. The pharmaceutical composition of clause 36, wherein the solid dispersion further comprises SLS.

38. A method of producing a pharmaceutical composition comprising providing an admixture comprising

a. a solid dispersion comprising substantially amorphous Compound 1;
b. a binder;
c. a glidant;
d. a surfactant;
e. a lubricant;
f. a disintegrant; and
g. a filler, and

compressing the admixture into a tablet having a dissolution of at least about 50% in about 30 minutes.

39. The method of clause 38, wherein the admixture is compressed to produce a tablet having a hardness of at least 5 Kp.

40. The method of clause 38, further comprising mixing the admixture until the admixture is substantially homogenous.

41. A method of administering a pharmaceutical composition comprising orally administering to a patient at least once per day at least one tablet comprising
a pharmaceutical composition comprising

a. solid dispersion comprising at least about 25 mg of substantially amorphous Compound 1;
b. a filler;
c. a binder;
d. a glidant;
e. a disintegrant;
f. a surfactant; and
g. a lubricant.

42. The method of clause 41, wherein the tablet comprising the pharmaceutical composition comprising the solid dispersion comprising substantially amorphous Compound 1, the filler, the binder, the glidant, the disintegrant, the surfactant, and the lubricant is orally administered to the patient once per day or about every 12 hours.

43. The method of clause 42, wherein the solid dispersion further comprises at least 75 mg of substantially amorphous Compound 1.

44. A method of administering a pharmaceutical composition comprising orally administering to a patient at least once per day at least one tablet comprising
a pharmaceutical composition comprising

a. a solid dispersion comprising at least about 25 mg of substantially amorphous Compound 1;
b. a filler;
c. a binder;
d. a glidant;
e. a disintegrant;
f. a surfactant, and

g. a lubricant.

45. A pharmaceutical composition consisting of a tablet comprising

a. a solid dispersion comprising from about 20 wt% to about 99 wt% of substantially amorphous Compound 1 by weight of the dispersion and a polymer selected from HPMCAS or PVP/VA;
b. from about 27 wt% to about 45 wt% of a filler comprising lactose;
c. from about 2.5 wt% to about 6.0 wt% of a disintegrant comprising sodium croscarmellose;
d. from about 2.0 wt% to about 0.3 wt% of a surfactant comprising sodium lauryl sulfate;
e. from about 20 wt% to about 45 wt% of a binder comprising microcrystalline cellulose;
f. from about 1.0 wt% to about 0.09 wt% of a glidant comprising colloidal silicon dioxide; and
g. from about 1.3 wt% to about 0.3 wt% of a lubricant comprising magnesium stearate.

46. A pharmaceutical composition comprising

a. about 15 wt% of a solid dispersion by weight of the composition, wherein the dispersion comprises about 50 wt% of substantially amorphous Compound 1 by weight of the dispersion, about 49.5 wt% of HPMCAS by weight of the dispersion, and about 0.5 wt% SLS by weight of the dispersion;
b. about 35 wt% of microcrystalline cellulose by weight of the composition;
c. about 43 wt% of lactose by weight of the composition; about 5 wt% of sodium croscarmellose by weight of the composition;
d. about 0.5 wt% of SLS by weight of the composition;
e. about 0.125 wt% of colloidal silicon dioxide by weight of the composition; and
f. about 0.5 wt% of magnesium stearate by weight of the composition.

47. A pharmaceutical composition comprising

a. about 31 wt% of a solid dispersion by weight of the composition, wherein the dispersion comprises about 50 wt% of substantially amorphous Compound 1 by weight of the dispersion, about 49.5 wt% of HPMCAS by weight of the dispersion, and about 0.5 wt% SLS by weight of the dispersion;
b. about 25 wt% of microcrystalline cellulose by weight of the composition;
c. about 38 wt% of lactose by weight of the composition;
d. about 5 wt% of sodium croscarmellose by weight of the composition;
e. about 0.5 wt% of SLS by weight of the composition;
f. about 0.125 wt% of colloidal silicon dioxide by weight of the composition; and
g. about 0.5 wt% of magnesium stearate by weight of the composition.

48. A pharmaceutical composition comprising

a. about 40 wt% of a solid dispersion by weight of the composition, wherein the dispersion comprises about 80 wt% of substantially amorphous Compound 1 by weight of the dispersion, about 19.5 wt% of PVP/VA by weight of the dispersion, and about 0.5 wt% SLS by weight of the dispersion;
b. about 27 wt% of microcrystalline cellulose by weight of the composition;
c. about 27 wt% of lactose by weight of the composition;
d. about 3 wt% of sodium croscarmellose by weight of the composition;
e. about 0.5 wt% of SLS by weight of the composition;
f. about 1 wt% of colloidal silicon dioxide by weight of the composition;
g. about 1 wt% of magnesium stearate by weight of the composition; and
h. about 0.4 wt% of colorant by weight of the composition.

49. A pharmaceutical composition comprising

a. about 40 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 27 wt% of microcrystalline cellulose by weight of the composition;
c. about 27 wt% of lactose by weight of the composition;
d. about 3 wt% of sodium croscarmellose by weight of the composition;

e. about 0.5 wt% of SLS by weight of the composition;
f. about 1 wt% of colloidal silicon dioxide by weight of the composition;
g. about 1 wt% of magnesium stearate by weight of the composition; and
h. about 0.4 wt% of colorant by weight of the composition.

50. A pharmaceutical composition comprising:

a. a solid dispersion comprising amorphous Compound 1 and a polymer;
b. a filler;
c. a disintegrant;
d. a surfactant;
e. a binder;
f. a glidant; and
g. a lubricant.

51. The pharmaceutical composition of clause 50, wherein the solid dispersion comprises substantially amorphous Compound 1 and a polymer, and the polymer comprises HPMC, HPMCAS, PVP/VA, PVP, methacrylic acid/methacrylate copolymer, HPC, or any combination thereof.

52. The pharmaceutical composition of clause 51, wherein the solid dispersion has a mean particle diameter of greater than about 5 $\mu$m.

53. The pharmaceutical composition of clause 51, wherein the solid dispersion has a bulk density of about 0.10 g/cc or greater.

54. The pharmaceutical composition of clause 51, wherein the solid dispersion comprises amorphous Compound 1, and Compound 1 is present in a concentration of at least 20 wt% by weight of the solid dispersion.

55. The pharmaceutical composition of clause 54, wherein the solid dispersion comprises 80 wt% or less of HPMCAS or PVP/VA.

56. The pharmaceutical composition of clause 55, wherein the solid dispersion comprises a surfactant.

57. The pharmaceutical composition of clause 56, wherein the solid dispersion comprises less than 10 wt% of surfactant by weight of solid dispersion.

58. The pharmaceutical composition of clause 57, wherein the solid dispersion comprises a surfactant, and the surfactant is SLS.

59. The pharmaceutical composition of clause 50, wherein the solid dispersion comprises from about 45 wt% to about 85 wt% of amorphous Compound 1, from about 0.45 wt% to about 0.55 wt% of SLS, and from about 14.45 wt% to about 55.55 wt% of HPMCAS or PVP/VA by weight of the solid dispersion.

60. The pharmaceutical composition of clause 50, wherein the solid dispersion comprises from about 40 wt% to about 60 wt% of amorphous Compound 1 by weight of the solid dispersion and from about 60 wt% to about 40 wt% of polymer by weight of the solid dispersion.

61. The pharmaceutical composition of clause 50, wherein the solid dispersion comprises from about 65 wt% to about 95 wt% of amorphous Compound 1 by weight of the solid dispersion and from about 45 wt% to about 5 wt% of polymer by weight of the solid dispersion.

62. The pharmaceutical composition of any of clauses 50-61, wherein the filler is lactose, sorbitol, cellulose, calcium phosphate, starch, sugar, or any combination thereof.

63. The pharmaceutical composition of any of clauses 50-62, wherein the filler is lactose and has a concentration of at least about 10 wt% by weight of the composition.

64. The pharmaceutical composition of any of clauses 50-63, wherein the disintegrant is sodium croscarmellose,

sodium starch glycolate, or a combination thereof.

65. The pharmaceutical composition of any of clauses 50-64, wherein the disintegrant is sodium croscarmellose and has a concentration of about 10 wt% or less by weight of the composition.

66. The pharmaceutical composition of any of clauses 50-65, wherein the surfactant is sodium lauryl sulfate, sodium stearyl fumarate, polyoxyethylene 20 sorbitan mono-oleate, or any combination thereof.

67. The pharmaceutical composition of any of clauses 50-66, wherein the surfactant is sodium lauryl sulfate and has a concentration of about 10 wt% or less by weight of the composition.

68. The pharmaceutical composition of any of clauses 50-67, wherein the binder is microcrystalline cellulose, dibasic calcium phosphate, sucrose, corn starch, modified cellulose, or any combination thereof.

69. The pharmaceutical composition of any of clauses 50-68, wherein the binder is microcrystalline cellulose and has a concentration of at least about 1 wt% by weight of the composition.

70. The pharmaceutical composition of any of clauses 50-69, wherein the glidant is colloidal silicon dioxide, talc, or a combination thereof.

71. The pharmaceutical composition of any of clauses 50-70, wherein the glidant is colloidal silicon dioxide and has a concentration of 2 wt% or less by weight of the composition.

72. The pharmaceutical composition of any of clauses 50-71, wherein the lubricant is magnesium stearate, stearic acid, hydrogenated oil, sodium stearyl fumarate, or any combination thereof.

73. The pharmaceutical composition of any of clauses 50-72, wherein the lubricant is magnesium stearate and has a concentration of about 2 wt% by weight of the composition.

74. The pharmaceutical composition of any of clauses 50-73, further comprising a colorant.

75. The pharmaceutical composition of any of clauses 50-74, wherein the colorant is a blue pigment having a concentration of less than 1 wt% by weight of the composition.

76. The pharmaceutical composition of any of clauses 31-37 and 45, wherein the tablet further comprises a coating.

77. The pharmaceutical composition of clause 76, wherein the coating comprises a colorant.

78. The pharmaceutical composition of clause 77, wherein the colorant comprises OPADRY® II.

79. The pharmaceutical composition of clauses 76-78, wherein the coating further comprises a wax coating.

80. The pharmaceutical composition of clause 79, wherein the wax coating comprises a Carnauba wax powder.

81. The pharmaceutical composition of clauses 76-80, wherein the tablet further comprises ink images or ink text printed on the coating.

82. A caplet shaped pharmaceutical tablet composition having a hardness of 9.5 Kp $\pm$ 15 percent, comprising

a. about 34 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 30 wt% of microcrystalline cellulose by weight of the composition;
c. about 30 wt% of lactose by weight of the composition;
d. about 3 wt% of sodium croscarmellose by weight of the composition;
e. about 0.5 wt% of SLS by weight of the composition;
f. about 1 wt% of colloidal silicon dioxide by weight of the composition; and
g. about 1 wt% of magnesium stearate by weight of the composition.

83. The caplet shaped pharmaceutical tablet composition of clause 82, wherein the tablet contains 150 mg of Compound 1.

84. The caplet shaped pharmaceutical tablet composition of clause 82, wherein the tablet contains 100 mg of Compound 1.

85. A caplet shaped pharmaceutical tablet composition having an initial hardness of 11 Kp ± 20 percent, comprising

a. about 40 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 30 wt% of microcrystalline cellulose by weight of the composition;
c. about 30 wt% of lactose by weight of the composition;
d. about 3 wt% of sodium croscarmellose by weight of the composition;
e. about 0.5 wt% of SLS by weight of the composition;
f. about 1 wt% of colloidal silicon dioxide by weight of the composition; and
g. about 1 wt% of magnesium stearate by weight of the composition.

86. The caplet shaped pharmaceutical tablet composition of clause 85, wherein the tablet contains 150 mg of Compound 1.

87. The caplet shaped pharmaceutical tablet composition of clause 85, wherein the tablet contains 100 mg of Compound 1.

88. A caplet shaped pharmaceutical tablet composition having an initial hardness of 11 Kp ± 20 percent, comprising

a. about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 30 wt% of microcrystalline cellulose by weight of the composition;
c. about 30.4 wt% of lactose by weight of the composition;
d. about 3 wt% of sodium croscarmellose by weight of the composition;
e. about 0.5 wt% of SLS by weight of the composition;
f. about 1 wt% of colloidal silicon dioxide by weight of the composition; and
g. about 1 wt% of magnesium stearate by weight of the composition.

89. The caplet shaped pharmaceutical tablet composition of clause 88, wherein the tablet contains 100 mg of Compound 1.

90. The caplet shaped pharmaceutical tablet composition of clause 88, wherein the tablet contains 150 mg of Compound 1.

91. The caplet shaped pharmaceutical tablet composition of clause 88, wherein the tablet includes a colorant coating and a printed logo or text.

92. The caplet shaped pharmaceutical tablet composition of clause 91, wherein the tablet includes a blue OPADRY@ II coating and a water or solvent based ink logo or text.

93. A caplet shaped pharmaceutical tablet composition having an initial hardness of between about 6 and 16 Kp, comprising

a. about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 30.5 wt% of microcrystalline cellulose by weight of the composition;
c. about 30.4 wt% of lactose by weight of the composition;
d. about 3 wt% of sodium croscarmellose by weight of the composition;
e. about 0.5 wt% of SLS by weight of the composition;

f. about 0.5 wt% of colloidal silicon dioxide by weight of the composition; and
g. about 1 wt% of magnesium stearate by weight of the composition.

94. The caplet shaped pharmaceutical tablet composition of clause 93, wherein the composition contains 100 mg of Compound 1.

95. The caplet shaped pharmaceutical tablet composition of clause 93, wherein the composition contains 150 mg of Compound 1.

96. The caplet shaped pharmaceutical tablet composition of clause 93, wherein the tablet further comprises a colorant coated, a wax coating, and a printed logo or text.

97. The caplet shaped pharmaceutical tablet composition of clause 96, wherein the tablet includes a blue OPADRY@ II coating and a water or solvent based ink logo or text.

98. The caplet of clause 96, wherein the wax coating comprises Carnauba wax.

99. The caplet of clause 96, wherein the ink for the printed logo or text is a solvent based ink.

100. A caplet shaped pharmaceutical tablet composition having an initial hardness of between about 9 and 21 Kp, comprising

a. about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion;
b. about 30.5 wt% of microcrystalline cellulose by weight of the composition;
c. about 30.4 wt% of lactose by weight of the composition;
d. about 3 wt% of sodium croscarmellose by weight of the composition;
e. about 0.5 wt% of SLS by weight of the composition;
f. about 0.5 wt% of colloidal silicon dioxide by weight of the composition; and
g. about 1 wt% of magnesium stearate by weight of the composition.

101. The caplet shaped pharmaceutical tablet composition of clause 100, wherein the tablet contains 150 mg of Compound 1.

102. The caplet shaped pharmaceutical tablet composition of clause 100, wherein the composition contains 100 mg of Compound 1.

103. The caplet shaped pharmaceutical tablet composition of clause 100, wherein the tablet further comprises a colorant coated, a wax coating, and a printed logo or text.

104. The caplet shaped pharmaceutical tablet composition of clause 103, wherein the tablet includes a blue OPADRY@ II coating and a water or solvent based ink logo or text.

105. The caplet of clause 103, wherein the wax coating comprises Carnauba wax.

106. The caplet of clause 103, wherein the ink for the printed logo or text is a solvent based ink.

107. A method of treating or lessening the severity of a disease in a patient comprising administering to said patient a pharmaceutical composition according to clauses 1- 37 and 45-101, wherein said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, Gorham's Syndrome, chloride channelopathies such as myotonia congenita (Thomson and Becker forms), Bartter's syndrome type III, Dent's disease, hyperekplexia, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

108. The method of clause 107, wherein said disease is cystic fibrosis.

109. The method of clause 108, wherein said patient has cystic fibrosis transmembrane receptor (CFTR) with a ΔF508 mutation.

110. The method of clause 109, wherein said patient has cystic fibrosis transmembrane receptor (CFTR) with a R117H mutation.

111. The method of clause 109, wherein said patient has cystic fibrosis transmembrane receptor (CFTR) with a G551D mutation.

## Claims

**1.** A pharmaceutical composition comprising a solid dispersion, wherein the solid dispersion comprises:

a) about 72 wt% to about 88 wt% of amorphous or substantially amorphous N-[2,4-Bis(1,1-dimethylethyl)-5-hydroxyphenyl]-1,4-dihydro-4-oxoquinoline-3-carboxamide (Compound 1) by weight of the dispersion, wherein substantially amorphous Compound 1 comprises less than 15% crystalline Compound 1; and
b) at least one polymer.

**2.** The pharmaceutical composition of claim 1, wherein the solid dispersion comprises about 80 wt% of amorphous or substantially amorphous Compound 1.

**3.** The pharmaceutical composition of any preceding claim, wherein the at least one polymer is independently selected from: hydroxypropylmethylcellulose (HPMC), hydroxypropylmethylcellulose acetate succinate (HPMCAS), vinylpyrrolidone/vinyl acetate copolymer (PVP/VA), polyvinylpyrrolidone (PVP), methacrylic acid/methacrylate copolymers, hydroxypropyl cellulose (HPC), or any combination thereof.

**4.** The pharmaceutical composition of any preceding claim, wherein the solid dispersion further comprises a surfactant.

**5.** The pharmaceutical composition of claim 4, wherein the solid dispersion comprises less than 10 wt% of surfactant by weight of the solid dispersion.

**6.** The pharmaceutical composition of claim 4 or 5, wherein the surfactant is selected from sodium lauryl sulfate (SLS), sodium stearyl fumarate (SSF), polyoxyethylene 20 sorbitan mono-oleate, or any combination thereof; preferably wherein the surfactant is SLS.

**7.** The pharmaceutical composition of any preceding claim, wherein the pharmaceutical composition further comprises:

a) 20 wt% to 45 wt% of microcrystalline cellulose by weight of the pharmaceutical composition;
b) 27 wt% to 45 wt% lactose by weight of the pharmaceutical composition;
c) 2.5 wt% to 6 wt% of sodium croscarmellose by weight of the pharmaceutical composition;
d) 0.3 wt% to 2 wt% of SLS by weight of the pharmaceutical composition;
e) 0.09 wt% to 1.0 wt% of colloidal silicon dioxide by weight of the composition; and/or

f) 0.30 wt% to 1.3 wt% of magnesium stearate by weight of the pharmaceutical composition.

**8.** The pharmaceutical composition of any preceding claim, wherein the solid dispersion comprises:

a) 150 mg of amorphous or substantially amorphous Compound 1; or
b) 100 mg of amorphous or substantially amorphous Compound 1.

**9.** The pharmaceutical composition of any preceding claim, comprising 34.1% of the sold dispersion by weight of the pharmaceutical composition.

**10.** The pharmaceutical composition of claim 1, further comprising:

a) a solid dispersion according to claim 1 in an amount ranging from 30% to 50% by weight of the pharmaceutical composition;
b) a filler, in an amount ranging from 25 wt% to 50 wt% by weight of the pharmaceutical composition;
c) a disintegrant, in an amount ranging from 1 wt% to 10 wt% by weight of the pharmaceutical composition;
d) a surfactant, in an amount ranging from 0.3 wt% to 2 wt% by weight of the pharmaceutical composition;
e) a binder, in an amount ranging from 5 wt% to 50 wt% by weight of the pharmaceutical composition;
f) a glidant, in an amount ranging from 0.05 wt% to 2 wt% by weight of the pharmaceutical composition; and
g) a lubricant, in an amount ranging from 0.1 wt% to 2 wt% by weight of the pharmaceutical composition.

**11.** The pharmaceutical composition of any preceding claim, comprising:

a) 34.1 wt% of a solid dispersion according to claim 1;
b) 30.5 wt% of microcrystalline cellulose by weight of the pharmaceutical composition;
c) 30.4 wt% of lactose by weight of the pharmaceutical composition;
d) 3 wt% of sodium croscarmellose by weight of the pharmaceutical composition;
e) 0.5 wt% of SLS by weight of the pharmaceutical composition;
f) 0.5 wt% of colloidal silicon dioxide by weight of the pharmaceutical composition; and
g) 1 wt% of magnesium stearate by weight of the composition.

**12.** The pharmaceutical composition of any preceding claim, wherein substantially amorphous Compound 1 comprises less than 5% crystalline Compound 1.

**13.** The pharmaceutical composition of any preceding claim, wherein the pharmaceutical composition is in tablet form.

**14.** The pharmaceutical composition of claim 13, wherein the tablet further comprises a coating.

**15.** The pharmaceutical composition of claim 14, wherein the tablet has a hardness of 98 N (10 kp) $\pm$ 20 percent.

**16.** The pharmaceutical composition of any preceding claim for use in a method for treating or lessening the severity of cystic fibrosis in a patient in need thereof.

**17.** The pharmaceutical composition for use according to claim 16, wherein said patient has:

a) cystic fibrosis transmembrane conductance regulator (CFTR) with a ΔF508 mutation; or
b) cystic fibrosis transmembrane conductance regulator (CFTR) with a R117H mutation; or
c) cystic fibrosis transmembrane conductance regulator (CFTR) with a G551D mutation.

**18.** The pharmaceutical composition for use according to claim 16, wherein the method comprises orally administering the pharmaceutical composition to the patient:

a) once per day; or
b) every 12 hours.

**19.** The pharmaceutical composition for use according to claim 16, wherein the method comprises orally administering the pharmaceutical composition:

a) concurrently with a high fat, high calorie CF meal or snack; or
b) 30 minutes after administering a high fat, high calorie CF meal or snack.

**20.** The pharmaceutical composition for use according to claim 16, wherein the method comprises administering the pharmaceutical composition concurrently with, prior to, or subsequent to one or more other desired therapeutics or medical procedures.

FIGURE 1

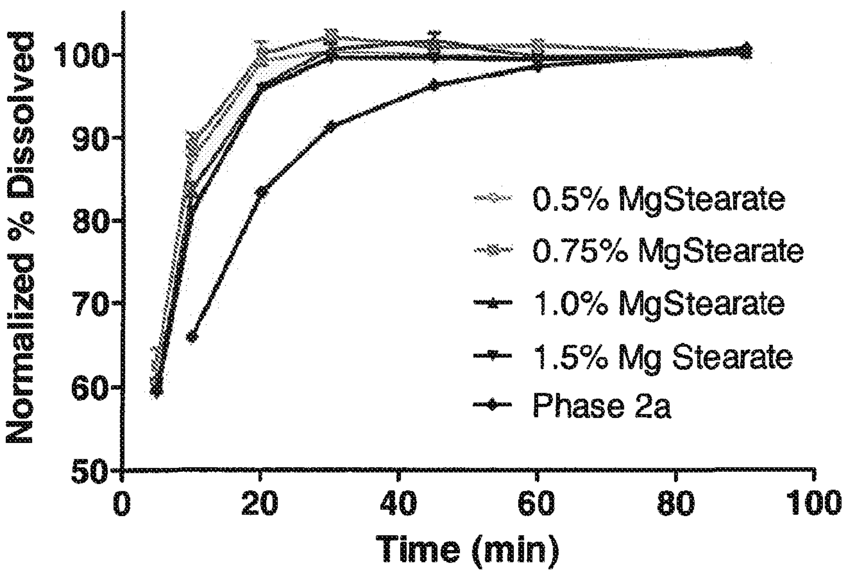
Normalized % Dissolved
100
90
80
70
60
50
0
20
40
60
80
100
Time (min)
0.5% MgStearate
0.75% MgStearate
1.0% MgStearate
1.5% Mg Stearate
Phase 2a

Europäisches Patentamt
European Patent Office
Office européen des brevets

# EUROPEAN SEARCH REPORT

Application Number
EP 20 21 1291

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,D | WO 2007/079139 A2 (VERTEX PHARMA [US]; HURTER PATRICIA [US] VERTEX PHARMA [US]; HURTER PA) 12 July 2007 (2007-07-12)<br>* page 28, paragraph 166 - page 42, paragraph 195; claims 11, 15, 18-34, 46, 75, 101, 130-131 *<br>----- | 1-20 | INV.<br>A61K9/20<br>A61K9/16<br>A61P3/00<br>A61P1/10<br>A61P3/06<br>A61P3/10<br>A61K9/28<br>A61P1/16<br>A61P11/00<br>A61P11/02<br>A61K31/47<br>A61P7/00<br>A61P7/10<br>A61P5/50<br>A61P5/14<br>A61P5/18<br>A61P37/08 |
| | | | TECHNICAL FIELDS SEARCHED (IPC) |
| | | | A61K |

1

The present search report has been drawn up for all claims

| Place of search | Date of completion of the search | Examiner |
|---|---|---|
| The Hague | 10 May 2021 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 21 1291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | | | A61P5/06<br>A61P27/02<br>A61P35/00 |
| | | | TECHNICAL FIELDS SEARCHED (IPC) |

The present search report has been drawn up for all claims

1

| Place of search | Date of completion of the search | Examiner |
|---|---|---|
| The Hague | 10 May 2021 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 21 1291

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2021

| Patent document cited in search report | Publication date | Patent family member(s) | Publication date |
|---|---|---|---|
| WO 2007079139 A2 | 12-07-2007 | AU 2006332726 A1 | 12-07-2007 |
| | | BR PI0620960 A2 | 29-11-2011 |
| | | CA 2635581 A1 | 12-07-2007 |
| | | CN 101384172 A | 11-03-2009 |
| | | CY 1118980 T1 | 10-01-2018 |
| | | DK 1993360 T3 | 22-05-2017 |
| | | DK 3219705 T3 | 14-04-2020 |
| | | EP 1993360 A2 | 26-11-2008 |
| | | EP 3219705 A1 | 20-09-2017 |
| | | EP 3708564 A1 | 16-09-2020 |
| | | ES 2624554 T3 | 14-07-2017 |
| | | ES 2790700 T3 | 28-10-2020 |
| | | HK 1125540 A1 | 14-08-2009 |
| | | HR P20170682 T1 | 14-07-2017 |
| | | HR P20200708 T1 | 24-07-2020 |
| | | HU E032813 T2 | 28-11-2017 |
| | | HU E049976 T2 | 30-11-2020 |
| | | JP 5409010 B2 | 05-02-2014 |
| | | JP 5734369 B2 | 17-06-2015 |
| | | JP 2009522278 A | 11-06-2009 |
| | | JP 2014012701 A | 23-01-2014 |
| | | JP 2015096539 A | 21-05-2015 |
| | | LT 1993360 T | 12-06-2017 |
| | | LT 3219705 T | 27-04-2020 |
| | | ME 03786 B | 20-04-2021 |
| | | PL 1993360 T3 | 31-08-2017 |
| | | PL 3219705 T3 | 10-08-2020 |
| | | PT 1993360 T | 25-05-2017 |
| | | PT 3219705 T | 20-05-2020 |
| | | SI 3219705 T1 | 31-08-2020 |
| | | US 2011064811 A1 | 17-03-2011 |
| | | US 2012214841 A1 | 23-08-2012 |
| | | US 2013317060 A1 | 28-11-2013 |
| | | US 2014242172 A1 | 28-08-2014 |
| | | US 2016229806 A1 | 11-08-2016 |
| | | US 2017231970 A1 | 17-08-2017 |
| | | US 2019070162 A1 | 07-03-2019 |
| | | US 2020352930 A1 | 12-11-2020 |
| | | WO 2007079139 A2 | 12-07-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61088704 B
- US 61088801 B
- US 61090096 B
- US 61146163 B
- US 61181527 B
- US 61183345 B
- US 20040006237 A
- WO 2007079139 PCT


### Non-patent literature cited in the description


- **GREGORY, R. J. et al.** *Nature,* 1990, vol. 347, 382-386
- **RICH, D. P. et al.** *Nature,* 1990, vol. 347, 358-362
- **RIORDAN, J. R. et al.** *Science,* 1989, vol. 245, 1066-1073
- **CUTTING, G. R. et al.** *Nature,* 1990, vol. 346, 366-369
- **DEAN, M. et al.** *Cell,* 1990, vol. 61 (863), 870
- **KEREM, B-S. et al.** *Science,* 1989, vol. 245, 1073-1080
- **KEREM, B-S et al.** *Proc. Natl. Acad. Sci. USA,* 1990, vol. 87, 8447-8451
- **QUINTON, P. M.** *FASEB J.,* 1990, vol. 4, 2709-2727
- **DALEMANS et al.** *Nature Lond.,* 1991, vol. 354, 526-528
- **PASYK ; FOSKETT.** *J. Cell. Biochem.,* 1995, vol. 270, 12347-50